(19) Europäisches Patentamt
European Patent Office
Office européen des brevets

(11) **EP 4 538 701 B1**

(12) **EUROPÄISCHE PATENTSCHRIFT**

(45) Veröffentlichungstag und Bekanntmachung des Hinweises auf die Patenterteilung:
**29.07.2026   Patentblatt 2026/31**

(21) Anmeldenummer: **24205214.0**

(22) Anmeldetag: **08.10.2024**

(51) Internationale Patentklassifikation (IPC):
*G01N 33/00* (2006.01)   *A61M 16/08* (2006.01)
*G01N 25/18* (2006.01)   *G01N 27/74* (2006.01)
*G01N 27/72* (2006.01)   *A61M 16/20* (2006.01)
*A61M 16/22* (2006.01)   *A61M 16/00* (2006.01)
*A61M 16/10* (2006.01)

(52) Gemeinsame Patentklassifikation (CPC):
(C-Sets verfügbar)
**A61M 16/0891; G01N 25/18; G01N 27/74;
G01N 33/0032;** A61M 16/208; A61M 16/22;
A61M 2016/0027; A61M 2016/1025;
A61M 2016/103; A61M 2016/1035; A61M 2230/432;
A61M 2230/437                              (Forts.)

(54) **ANORDNUNG UND VERFAHREN ZUM MESSEN DER JEWEILIGEN KONZENTRATION VON DREI GASBESTANDTEILEN IN EINER GASPROBE**

ARRANGEMENT AND METHOD FOR MEASURING THE RESPECTIVE CONCENTRATION OF THREE GAS COMPONENTS IN A GAS SAMPLE

DISPOSITIF ET PROCÉDÉ DE MESURE DE LA CONCENTRATION RESPECTIVE DE TROIS COMPOSANTS GAZEUX DANS UN ÉCHANTILLON DE GAZ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **13.10.2023   DE 102023128115**

(43) Veröffentlichungstag der Anmeldung:
**16.04.2025   Patentblatt 2025/16**

(73) Patentinhaber: **Drägerwerk AG & Co. KGaA 23558 Lübeck (DE)**

(72) Erfinder:
• **Jahns, Robert**
  **23558 Lübeck (DE)**
• **Stark, Hartmut**
  **23558 Lübeck (DE)**
• **Hansmann, Hans-Ullrich**
  **23558 Lübeck (DE)**
• **Dicks, Bernd-Michael**
  **23558 Lübeck (DE)**
• **Kähler, Hendrik**
  **23558 Lübeck (DE)**

(56) Entgegenhaltungen:
DE-A1- 102010 014 883     DE-A1- 102021 126 106
GB-A- 2 355 806

(52) Gemeinsame Patentklassifikation (CPC): (Forts.)

C-Sets
A61M 2230/432, A61M 2230/005;
A61M 2230/437, A61M 2230/005

**Beschreibung**

Die Erfindung betrifft eine Sensor-Anordnung und ein Mess-Verfahren, um die jeweilige Konzentration von drei Gasbestandteilen einer Gasprobe zu messen.

Die Aufgabe, die jeweilige Konzentration von mehreren Gasbestandteilen in einer Gasprobe zu messen, tritt beispielsweise bei der künstlichen Beatmung eines Patienten auf. Ein Beatmungsgerät fördert ein atembares Gasgemisch zu einer patientenseitigen Koppeleinheit, beispielsweise zu einer Atemmaske oder einem Tubus, wobei die patientenseitige Koppeleinheit mit dem Patienten verbunden ist. Das atembare Gasgemisch enthält Sauerstoff und in einer Ausgestaltung mindestens ein Narkosemittel (Anästhesiegas).

Ein Verfahren und eine Vorrichtung, um die Sauerstoff-Konzentration und die Narkosemittel-Konzentration zu messen, wird in DE 10 2021 126 106 A1 beschrieben. In einer Messkammer wird ein Magnetfeld mit einer oszillierenden Magnetfeldstärke erzeugt. Die Wärmeleitfähigkeit und die magnetisch modulierte Wärmeleitfähigkeit des atembaren Gasgemischs werden gemessen. Von der Tatsache, dass Sauerstoff ein paramagnetisches Gas ist, wird Gebrauch gemacht. In GB 2 355 806 A wird ein ähnlicher Sensor beschrieben.

Der Erfindung liegt die Aufgabe zugrunde, eine Sensor-Anordnung und ein Mess-Verfahren bereitzustellen, welche die jeweilige Konzentration von drei Gasbestandteilen einer Gasprobe zu messen vermögen, und zwar mit einer höheren Zuverlässigkeit als bekannte Sensor-Anordnungen und Mess-Verfahren.

Die Aufgabe wird durch eine Sensor-Anordnung mit den Merkmalen des Anspruchs 1 und durch ein Mess-Verfahren mit den Merkmalen des Anspruchs 6 gelöst. Vorteilhafte Ausgestaltungen des erfindungsgemäßen Sensor-Anordnung sind, soweit sinnvoll, auch vorteilhafte Ausgestaltungen des erfindungsgemäßen Mess-Verfahrens und umgekehrt.

Die erfindungsgemäße Sensor-Anordnung und das erfindungsgemäße Mess-Verfahren vermögen die jeweilige Konzentration (den jeweiligen Anteil) von drei verschiedenen Gasbestandteilen einer Gasprobe zu messen. Bevorzugt wird unter "der Konzentration" der jeweilige Anteil in Vol.-% verstanden, die Konzentration kann auch der Anteil in Gew.-% sein, also der Mengen-Anteil. Ein Gasbestandteil der Gasprobe ist ein paramagnetisches Gas, insbesondere Sauerstoff.

Anmerkung: Ein paramagnetisches Gas hat folgende Eigenschaften: Die Atome oder Moleküle des Gases besitzen permanente magnetische Eigenschaften (ein magnetisches Moment) und richten sich in einem Magnetfeld aus.

Die jeweilige Dichte und die jeweilige Wärmeleitfähigkeit jedes der drei Gasbestandteile sind in rechnerauswertbarer Form vorgegeben. Beispielsweise hat die Sensor-Anordnung wenigstens zeitweise Lesezugriff auf einen Datenspeicher, in dem die Dichten und Wärmeleitfähigkeiten abgespeichert sind.

Die Sensor-Anordnung umfasst eine Messkammer, einen Wärmeleitfähigkeits-Sensor und einen Dichtesensor. Die Messkammer vermag eine Gasprobe aufzunehmen. Der Wärmeleitfähigkeits-Sensor vermag die Wärmeleitfähigkeit der Gasprobe in der Messkammer zu messen. Der Dichtesensor vermag die Dichte der Gasprobe zu messen. Das erfindungsgemäße Mess-Verfahren wird unter Verwendung einer solchen Sensor-Anordnung durchgeführt und umfasst die Schritte, dass die Wärmeleitfähigkeit und die Dichte der Gasprobe gemessen werden.

Anmerkung: Die Formulierung, dass ein Sensor eine physikalische Größe misst, bedeutet folgendes: Der Sensor misst direkt die physikalische Größe oder mindestens eine andere physikalische Größe, wobei die oder jede andere physikalische Größe mit der zu messenden physikalischen Größe korreliert. Die oder jede andere physikalische Größe ist somit ein Maß für die zu messende physikalische Größe.

Das erfindungsgemäße Mess-Verfahren umfasst weiterhin die folgenden Schritte, und die erfindungsgemäße Sensor-Anordnung ist dazu ausgestaltet, die folgenden Schritte durchzuführen:

- Die Gasprobe wird in die Messkammer gefördert oder geleitet.
- An die Messkammer wird ein Magnetfeld mit einer oszillierenden Magnetfeldstärke angelegt.
- Die magnetisch modulierte Wärmeleitfähigkeit der Gasprobe in der Messkammer wird gemessen.

Unter der "magnetisch modulierten Wärmeleitfähigkeit" der Gasprobe wird derjenige Anteil der Wärmeleitfähigkeit verstanden, der abhängig von der Magnetfeldstärke oszilliert. Diese magnetisch modulierte Wärmeleitfähigkeit hängt wesentlich, aber in der Regel nicht ausschließlich, von der Konzentration des paramagnetischen Gases in der Gasprobe ab. Möglich ist, dass als Maß für die (gesamte) Wärmeleitfähigkeit der Gasprobe diejenige Wärmeleitfähigkeit gemessen wird, welche die Gasprobe in der Messkammer aufweist. Möglich ist, dass derselbe Wärmeleitfähigkeits-Sensor sowohl die Wärmeleitfähigkeit als auch die magnetisch modulierte Wärmeleitfähigkeit misst, also zwei verschiedene Signale liefert, die voneinander unabhängig sein können. Möglich ist auch, dass zwei verschiedene Wärmeleitfähigkeits-Sensoren verwendet werden.

Die drei Gasbestandteil-Konzentrationen werden unter Verwendung folgender Informationen ermittelt:

- der vorgegebenen Dichten der drei Gasbestandteile,
- der vorgegebenen Wärmeleitfähigkeiten der drei Gasbestandteile,

- der gemessenen Wärmeleitfähigkeit der Gasprobe,
- der gemessenen magnetisch modulierten Wärmeleitfähigkeit der Gasprobe und
- der gemessenen Dichte der Gasprobe,
- optional zusätzlich der Temperatur der Gasprobe,
- optional zusätzlich der Temperatur in der Messkammer und
- optional zusätzlich der Druck, unter dem die Gasprobe steht, bevorzugt dem Druck in der Messkammer.

Erfindungsgemäß werden drei verschiedene Eigenschaften der Gasprobe gemessen, nämlich die Wärmeleitfähigkeit, die magnetisch modulierte Wärmeleitfähigkeit und die Dichte. In aller Regel hängen diese drei Eigenschaften der Gasprobe von den gesuchten Konzentrationen der drei Gasbestandteile in der Gasprobe ab, hängen aber nicht voneinander ab. Insbesondere verändern sich diese drei Eigenschaften in der Regel unabhängig voneinander. Mithilfe dieser drei voneinander unabhängigen und durch Messungen ermittelten und daher bekannten Eigenschaften der Gasprobe lassen sich drei Unbekannte ermitteln, nämlich die drei gesuchten Konzentrationen der drei Gasbestandteile.

Die Erfindung setzt nicht voraus, dass die Gasprobe nur aus diesen drei Gasbestandteilen besteht. Vielmehr kann die Gasprobe mindestens einen weiteren Gasbestandteil umfassen. Um dessen Konzentration zu ermitteln, lässt sich in vielen Fällen die Tatsache ausnutzen, dass die Summe der Konzentrationen der Gasbestandteile, gemessen in Vol.-% oder in Gew.-%, 100% ergibt.

In manchen Anwendungen wird vor allem die Konzentration des paramagnetischen Gases benötigt, wobei das paramagnetische Gas insbesondere Sauerstoff ist. Manche bekannte Sensor-Anordnungen und Mess-Verfahren vermögen ebenfalls die Sauerstoff-Konzentration in einer Gasprobe zu messen, weisen aber eine relativ hohe unerwünschte Querempfindlichkeit gegenüber weiteren Bestandteilen der Gasprobe auf. Eine solche Querempfindlichkeit kann zu falschen Messergebnissen führen und ist daher unerwünscht. Die erfindungsgemäße Sensor-Anordnung und das erfindungsgemäße Mess-Verfahren sind hingegen deutlich weniger querempfindlich, idealerweise überhaupt nicht querempfindlich, gegenüber weiteren Gasbestandteilen in der Gasprobe.

Dank der Erfindung ist es in vielen Fällen nicht erforderlich, ein Referenzgas mit einer bekannten Zusammensetzung bereitzustellen und für die Messung zu verwenden, während die Sensor-Anordnung und das Mess-Verfahren die Gasbestandteil-Konzentrationen messen.

Bekannt geworden sind elektrochemische Sensoren. In einem elektrochemischen Sensor läuft ein chemischer Prozess ab, der von der Konzentration von Sauerstoff und / oder einem sonstigen Gasbestandteil in der Gasprobe abhängt. Ein Detektionsgrößen-Sensor des elektrochemischen Sensors misst eine elektrische Detektionsgröße, die mit der gesuchten Sauerstoff-Konzentration korreliert. In manchen Fällen haben solche elektrochemischen Sensoren den Nachteil, dass sie eine Chemikalie erfordern, welche sich im Laufe der Zeit verbraucht und daher von Zeit zu Zeit ergänzt werden muss. Außerdem kann eine Chemikalie durch Umgebungseinflüsse, insbesondere durch Umgebungswärme, verändert werden oder verdunsten. Die Erfindung kommt ohne eine Chemikalie aus und vermeidet daher diese Nachteile.

In einer Anwendung ist der paramagnetische Gasbestandteil Sauerstoff. Die beiden weiteren Gasbestandteile, deren Konzentrationen erfindungsgemäß gemessen werden, sind bei dieser Anwendung ein Narkosemittel (ein Anästhesiegas) oder Kohlendioxid sowie Argon. Das Edelgas Argon kommt in der Umgebungsluft mit einer bekannten Konzentration vor. Die Konzentration von Argon in der Gasprobe ist aber in vielen Fällen höher als die bekannte Konzentration von Argon in der Umgebungsluft. Diese Situation tritt insbesondere bei der folgenden Anwendung auf: Die Gasprobe stammt aus einem atembaren Gasgemisch, welches zur künstlichen Beatmung eines Patienten verwendet wird. Das Gasgemisch wird unter Verwendung von Umgebungsluft hergestellt, hat aber einen höheren Sauerstoffanteil als Umgebungsluft. Um das Gasgemisch aus Umgebungsluft zu erzeugen, wird ein sogenannter Konzentrator verwendet. Falls ein Gasgemisch mit einem höheren Sauerstoffanteil als Umgebungsluft mittels eines Konzentrators erzeugt wird, weist das erzeugte Gasgemisch in der Regel auch eine höhere Konzentration von Argon als die Umgebungsluft auf. Diese höhere Konzentration wird bei der gerade beschriebenen Anwendung der Erfindung gemessen.

Bevorzugt vermag die Sensor-Anordnung gleichzeitig sowohl die Wärmeleitfähigkeit als auch die magnetisch modulierte Wärmeleitfähigkeit einer Gasprobe in derselben Messkammer zu messen. In einer Ausgestaltung umfasst die Sensor-Anordnung ein Heizelement und vermag das Heizelement zu erhitzen, insbesondere dadurch, dass eine elektrische Spannung an das Heizelement angelegt wird und bewirkt wird, dass ein elektrischer Strom durch einen elektrischen Heizdraht des Heizelements fließt. Wenn das Heizelement erhitzt ist, führt es einer Gasprobe in der Messkammer Wärmeenergie zu. Bevorzugt reagiert dieses Heizelement nicht chemisch mit der Gasprobe in der Messkammer.

Erfindungsgemäß wird an die Messkammer ein Magnetfeld mit einer oszillierenden Magnetfeldstärke angelegt. In der Messkammer werden die Wärmeleitfähigkeit und die magnetisch modulierte Wärmeleitfähigkeit der Gasprobe gemessen. Bevorzugt wird eine elektrische Detektionsgröße gemessen. Diese elektrische Detektionsgröße korreliert mit der Wärmeleitfähigkeit der Gasprobe in der Messkammer. Eine elektrische oder elektronische Filterung der elektrischen Detektionsgröße wird durchgeführt. Diese Filterung liefert zwei Signale, nämlich ein oszillierendes Signal und ein weiteres Signal. Das oszillierende Signal oszilliert abhängig von der oszillierenden Magnetfeldstärke. Der zeitliche Verlauf des weiteren Signals hängt idealerweise nicht von der oszillierenden Magnetfeldstärke ab. Das oszillierende Signal wird als das oder als ein Maß für die magnetisch modulierte Wärmeleitfähigkeit verwendet. Das oszillierende Signal korreliert also mit der Konzentration des paramagnetischen Gases in der Gasprobe. Das weitere Signal wird als das oder ein Maß für die Wärmeleitfähigkeit der Gasprobe verwendet. Es korreliert mit der Wärmeleitfähigkeit der Gasprobe.

Diese Ausgestaltung führt in vielen Fällen zu einer besonders kompakten Sensor-Anordnung. In derselben Messkammer werden mit Hilfe desselben Magnetfelderzeugers und desselben erhitzten Heizelements zwei voneinander unabhängige Eigenschaften der Gasprobe gemessen, nämlich die Wärmeleitfähigkeit und die magnetisch modulierte Wärmeleitfähigkeit.

Verschiedene Ausgestaltungen sind möglich, wie die Dichte der Gasprobe gemessen wird. In einer Ausgestaltung wird ein mechanischer oder elektronischer Biegeschwinger (flexural resonator) verwendet. Dieser Biegeschwinger umfasst einen Schwingkörper. Im Falle eines mechanischen Biegeschwingers ist oder umfasst dieser Schwingkörper eine beweglich gelagerte Fluidführungseinheit. Unter einer "Fluidführungseinheit" wird ein Bauteil verstanden, welches ein Fluid entlang einer Trajektorie leitet, wobei diese Trajektorie durch die Konstruktion und Anordnung des Bauteils vorgegeben ist. Ein Faltenschlauch, ein glatter Schlauch und eine Röhre sind Beispiele für eine Fluidführungseinheit. Die Fluidführungseinheit umfasst nicht notwendigerweise eine Fördereinheit. Im Falle eines elektronischen Biegeschwingers ist dieser Schwingkörper bevorzugt ein Schwingquarz.

Die Gasprobe wird durch die Fluidführungseinheit des Biegeschwingers hindurchgeleitet oder am Schwingquarz vorbeigeleitet. Der Biegeschwinger vermag den Schwingkörper (Fluidführungseinheit oder Schwingquarz) in Schwingungen zu versetzen, während sich ein Teil der Gasprobe in der Fluidführungseinheit befindet oder am Schwingkörper vorbeigeleitet wird oder den Schwingkörper umgibt. Die Eigenfrequenz des schwingenden Schwingkörpers wird gemessen. Diese gemessene Eigenfrequenz korreliert mit der Masse der Gasprobe.

Im Falle eines mechanischen Biegeschwingers korreliert die Eigenfrequenz mit der Masse der gefüllten Fluidführungseinheit. In der Regel ist diese Masse die Summe aus der Masse der leeren Fluidführungseinheit und der gesuchten Masse der Gasprobe in der Fluidführungseinheit. Die Masse der leeren Fluidführungseinheit ist durch die Konstruktion des Biegeschwingers bekannt und verändert sich bei einer Messung nicht. Das Volumen der Fluidführungseinheit ist ebenfalls durch die Konstruktion bekannt und bleibt konstant. Aus der gemessenen Eigenfrequenz sowie der Masse und dem Volumen der leeren Fluidführungseinheit lässt sich die gesuchte Dichte der Gasprobe herleiten.

Das Prinzip eines solchen Biegeschwingers ist seit langem bekannt, und zuverlässige Biegeschwinger zur Dichtemessung stehen zur Verfügung.

In einer Ausgestaltung wird die Konzentration mindestens eines vierten Gasbestandteils der Gasprobe direkt gemessen. Die jeweilige Konzentration des oder jedes vierten Gasbestandteils der Gasprobe wird für den Schritt, die jeweilige Konzentration der drei Gasbestandteile zu ermitteln, verwendet. Diese Ausgestaltung erhöht in vielen Fällen die Zuverlässigkeit, mit der die drei Konzentrationen der drei Gasbestandteile ermittelt werden.

In einer Realisierungsform dieser Ausgestaltung wird die Konzentration des oder mindestens eines vierten Gasbestandteils durch einen photo-elektrischen Sensor gemessen, der das folgende wohlbekannte Prinzip anwendet:

- Eine Strahlungsquelle emittiert elektromagnetische Strahlung oder auch Schall oder Ultraschall in eine Messstrecke oder Messkammer hinein, und zwar ausreichend breitbandig. Die Gasprobe durchfließt diese Messstrecke oder befindet sich in dieser Messkammer. Die Messkammer kann die gerade beschriebene Messkammer mit der Magnetfeldstärke sein oder eine räumlich beabstandete weitere Messstrecke oder Messkammer.

- Die elektromagnetische Strahlung oder der Schall durchdringen die Messstrecke oder die Messkammer. Wenigstens ein Teil trifft auf einen Detektor auf.

- Die Gasprobe in der Messstrecke oder Messkammer absorbiert einen Teil der Strahlung oder des Schalls. Dadurch wird die Intensität der Strahlung oder des Schalls in einem Wellenlängenbereich reduziert.

- Ein Detektor misst die Intensität der auftreffenden Strahlung oder des auftreffenden Schalls. Diese Intensität korreliert mit der gesuchten Konzentration des vierten Gasbestandteils.

- Der Detektor generiert abhängig von der gemessenen Intensität ein Signal. Dieses Signal umfasst eine Information über die Intensität und daher ebenfalls über die Konzentration des vierten Gasbestandteils.

Viele Gase weisen einen charakteristischen spektralen Verlauf der Absorption auf, also eine charakteristische Abhängigkeit des Absorptionsgrads von der Wellenlänge der elektromagnetischen Strahlung oder des Schalls, welche / welcher dieses Gas durchdringt. In der Regel lässt sich die Konzentration des vierten Gasbestandteils unabhängig von den gesuchten Konzentrationen der drei Gasbestandteile messen.

Erfindungsgemäß werden drei Eigenschaften der Gasprobe gemessen, um Werte für drei Unbekannte zu ermitteln, wobei die drei Unbekannten die drei gesuchten Konzentrationen der drei Gasbestandteile in der Gasprobe sind. In einer Ausgestaltung werden drei funktionale Zusammenhänge, drei Abstandsmaße und eine Zielfunktion vorgegeben. Bevorzugt hat die erfindungsgemäße Sensor-Anordnung wenigstens zeitweise Lesezugriff auf einen Datenspeicher, in dem in einer rechnerauswertbaren Form die Zielfunktion, die Abstandsmaße und die funktionalen Zusammenhänge abgespeichert sind.

Der erste funktionale Zusammenhang beschreibt die Wärmeleitfähigkeit der Gasprobe als Funktion der drei gesuchten Gasbestandteil-Konzentrationen. Der zweite funktionale Zusammenhang beschreibt die magnetisch modulierte Wärmeleitfähigkeit der Gasprobe als Funktion der drei Gasbestandteil-Konzentrationen. Der dritte funktionale Zusammenhang beschreibt die Dichte der Gasprobe als Funktion der drei Gasbestandteil-Konzentrationen. Alle drei funktionalen Zusammenhänge hängen also von den drei unbekannten und gesuchten Gasbestandteil-Konzentrationen ab.

Das erste Abstandsmaß beschreibt ein Maß für einen Abstand zwischen der gemessenen Wärmeleitfähigkeit der Gasprobe und der Wärmeleitfähigkeit, die mit Hilfe des ersten funktionalen Zusammenhangs hergeleitet wird. Das zweite Abstandsmaß beschreibt ein Maß für einen Abstand zwischen der gemessenen magnetisch modulierten Wärmeleitfähigkeit der Gasprobe und der magnetisch modulierten Wärmeleitfähigkeit, die mit Hilfe des zweiten funktionalen Zusammenhangs hergeleitet wird. Das dritte Abstandsmaß beschreibt ein Maß für einen Abstand zwischen der gemessenen Dichte der Gasprobe und der Dichte, die mit Hilfe des dritten funktionalen Zusammenhangs hergeleitet wird. Bevorzugt ist jedes Abstandsmaß das Quadrat der jeweiligen Differenz, alternativ der Betrag der Differenz oder eine sonstige Funktion, die umso größer ist, je größer der Betrag der Differenz ist.

Die Zielfunktion ist eine Aggregation dieser drei Abstandsmaße. Bevorzugt ist die Zielfunktion ein gewichtetes Mittel der drei Abstandsmaße. Die Gewichtsfaktoren kompensieren beispielsweise unterschiedliche Größenordnungen der drei Eigenschaften Wärmeleitfähigkeit, magnetisch modulierte Wärmeleitfähigkeit und Dichte. Möglich ist auch, dass die Gewichtsfaktoren zusätzlich oder stattdessen unterschiedliche Genauigkeits-Anforderungen an die gesuchten Gasbestandteil-Konzentrationen berücksichtigen.

Die drei gemessenen Werte für die Gasprobe, also die gemessene Wärmeleitfähigkeit, die magnetisch modulierte Wärmeleitfähigkeit und die Dichte der Gasprobe, werden in die Zielfunktion eingesetzt. Nach dem Einsetzen hängt die Zielfunktion von drei Unbekannten ab, nämlich von den drei gesuchten Gasbestandteil-Konzentrationen. Die Zielfunktion wird numerisch minimiert. Dies bedeutet: Verschiedene Wertetripel für die drei Gasbestandteil-Konzentrationen werden probeweise in die Zielfunktion eingesetzt. Jedes Wertetripel ordnet jeder der drei Konzentrationen jeweils einen Wert zu. Das oder ein Wertetripel, das zu einem minimalen Wert der Zielfunktion führt, liefert die drei gesuchten Gasbestandteil-Konzentrationen. Idealerweise liefert das minimierende Wertetripel den Wert null für die Zielfunktion. In der Praxis tritt jeweils ein Abstand zwischen der gemessenen Eigenschaft und der gemäß des funktionalen Zusammenhangs vorhergesagten Eigenschaft auf. Bevorzugt wird ein iteratives Minimierungsverfahren angewendet. Möglich ist, dass die Zielfunktion mehrere Minima aufweist.

Möglich ist, dass in mindestens einem funktionalen Zusammenhang zusätzlich die Konzentration eines vierten Gasbestandteils auftritt. In einer Realisierungsform misst ein Sensor direkt die Konzentration dieses vierten Gasbestandteils, beispielsweise mit dem weiter oben beschriebenen photo-elektrischen Messprinzip umfassend eine Strahlungsquelle und einen Detektor. In einer anderen Realisierungsform wird für die Konzentration des vierten Gasbestandteils ein Standardwert vorgegeben. Möglich ist, dass es einen weiteren, also einen fünften Gasbestandteil gibt, dessen Konzentration gemessen wird oder für den ein Standardwert vorgegeben wird.

Die Erfindung betrifft weiterhin eine Beatmungs-Anordnung und ein Beatmungs-Verfahren zur künstlichen Beatmung eines Patienten. Die Beatmungs-Anordnung umfasst

- ein medizinisches Gerät, insbesondere ein Beatmungsgerät,
- eine patientenseitige Koppeleinheit,
- ein Beatmungs-Steuergerät und
- eine erfindungsgemäße Sensor-Anordnung.

Das Beatmungs-Verfahren wird unter Verwendung einer solchen Beatmungs-Anordnung durchgeführt.

Der Patient ist mit der patientenseitigen Koppeleinheit verbunden oder lässt sich wenigstens zeitweise mit einer patientenseitigen Koppeleinheit verbinden. Die patientenseitige Koppeleinheit umfasst insbesondere eine Atemmaske und / oder einen Tubus und / oder einen Katheter. Die Beatmungs-Anordnung vermag ein atembares Gasgemisch vom medizinischen Gerät zur patientenseitigen Koppeleinheit zu leiten und / oder zu fördern. Das Gasgemisch enthält Sauerstoff und in einer Ausgestaltung mindestens ein Narkosemittel und / oder ein Medikament. Möglich ist, dass ein Beatmungskreislauf hergestellt ist. Das vom Patienten ausgeatmete Gasgemisch fließt im Beatmungskreislauf zurück zum medizinischen Gerät.

Die Konzentration mindestens eines Gasbestandteils des atembaren Gasgemischs, insbesondere die Konzentration von Sauerstoff, soll in einem vorgegebenen Sollbereich liegen. Möglich ist, dass dieser Sollbereich zeitlich veränderlich ist. Möglich ist auch, dass für mindestens zwei Gasbestandteile jeweils ein Sollbereich vorgegeben ist.

Die Beatmungs-Anordnung ist dazu ausgestaltet, die folgenden Schritte durchzuführen, und das Beatmungs-Verfahren umfasst die folgenden Schritte:

- Aus dem Gasgemisch, das zur patientenseitigen Koppeleinheit gefördert wird, wird eine Gasprobe abgezweigt.
- Die abgezweigte Gasprobe wird zur Sensor-Anordnung geleitet.
- Die Sensor-Anordnung misst mindestens die Konzentration des oder jedes Gasbestandteils, für den ein Sollbereich vorgegeben ist, in der abgezweigten Gasprobe, optional die Konzentration mindestens eines weiteren Gasbestandteils.

Das Beatmungs-Steuergerät führt eine Regelung (closed-loop control) oder auch eine Steuerung (open-loop control) durch. Die Regelgröße ist die tatsächliche Konzentration mindestens eines Gasbestandteils im Gasgemisch, das zur patientenseitigen Koppeleinheit geführt wird. Das Regelungsziel (control gain) bei der Regelung ist, dass die Regelgröße, also die tatsächliche Gasbestandteil-Konzentration, im vorgegebenen Sollbereich liegt. Für die Regelung verwendet das Beatmungs-Steuergerät die Gasbestandteil-Konzentration in der abgezweigten Gasprobe, wobei die Sensor-Anordnung diese Konzentration gemessen hat.

Im Folgenden wird die Erfindung anhand eines Ausführungsbeispiels beschrieben. Hierbei zeigt

Figur 1 schematisch einen Beatmungskreislauf zur künstlichen Beatmung eines Patienten;

Figur 2 schematisch eine erste Ausführungsform eines Sensors, der die Konzentration von Sauerstoff und einem weiteren Gas zu messen vermag;

Figur 3 schematisch eine zweite Ausführungsform eines solchen Sensors;

Figur 4 die Messeinheit des Sensors von Figur 3;

Figur 5 eine beispielhafte Auswerteschaltung des Sensors von Figur 2 und von Figur 3;

Figur 6 einen mechanischen Biegeschwinger, mit dem die Dichte der Gasprobe gemessen wird;

Figur 7 einen elektronischen Biegeschwinger in Form eines Schwingquarzes, mit dem die Dichte der Gasprobe gemessen wird;

Figur 8 beispielhaft die magnetisch modulierte Wärmeleitfähigkeit $\lambda_{2f}$ eines Gasgemischs als Funktion der Sauerstoffkonzentration.

Im Ausführungsbeispiel wird die erfindungsgemäße Sensor-Anordnung für die künstliche Beatmung eines Patienten verwendet. Der Patient wird mit einem atembaren Gasgemisch versorgt, welches Sauerstoff und mindestens ein Narkosemittel (Anästhesiemittel) umfasst. Der Patient ist daher narkotisiert oder wenigstens sediert.

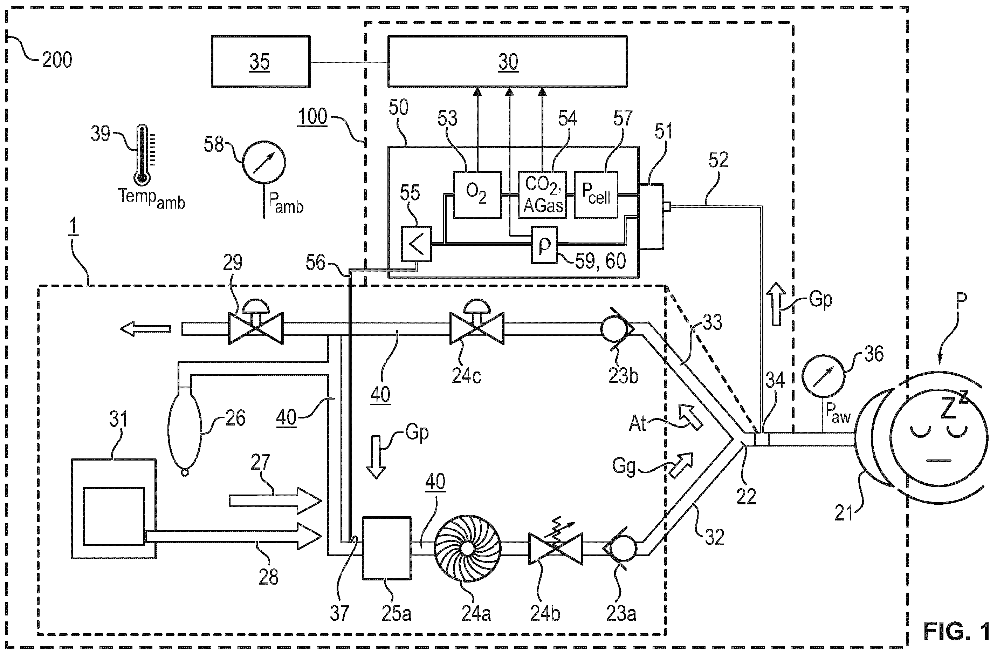

Figur 1 zeigt schematisch eine Beatmungs-Anordnung 200 mit einem schematisch gezeigten Beatmungsgerät 1, wobei die Beatmungs-Anordnung 200 einen Patienten P künstlich zu beatmen vermag. Vom Patienten P wird lediglich dessen Gesicht schematisch gezeigt. Das Beatmungsgerät 1 hält im gezeigten Beispiel einen Beatmungskreislauf 40 aufrecht. Im gezeigten Beispiel ist das Beatmungsgerät 1 als Anästhesiegerät ausgestaltet, und der Patient P ist narkotisiert oder wenigstens sediert.

Im Beatmungskreislauf 40 wird dem Patienten P ein atembares Gasgemisch Gg zugeführt. Dieses Gasgemisch Gg umfasst im Ausführungsbeispiel Sauerstoff ($O_2$), mindestens ein Narkosemittel und optional ein Trägergas für ein Narkosemittel, z.B. Lachgas ($N_2O$) und optional zusätzlich ein Medikament. Ein Narkosemitteldosierer 31 erzeugt einen Fluidstrom 28 mit dampfförmigem Narkosemittel und einem Trägergas. Der Fluidstrom 28 wird in den Beatmungskreislauf 40 eingespeist. Außerdem wird wenigstens zeitweise ein Fluidstrom 27 von Frischluft oder von sonstigem Frischgas eingespeist. Zumindest solange im Beatmungskreislauf 40 kein zu großer Überdruck herrscht, bleibt das vom Patienten P ausgeatmete Gasgemisch im Beatmungskreislauf 40, so dass kein Narkosemittel in die Umgebung gelangt.

Zwangsläufig enthält das Gasgemisch At, welches vom Patienten P ausgeatmet wird und durch den Beatmungskreislauf 40 fließt, Kohlendioxid ($CO_2$). Ein Kohlendioxidabsorber ($CO_2$-Absorber) 25a vermag Kohlendioxid aus dem Beatmungskreislauf 40 zu absorbieren. Außerdem können im atembaren Gasgemisch Gg weitere Bestandteile vorhanden sein, die auch in der Umgebungsluft auftreten, allerdings möglicherweise mit einer anderen Konzentration als in Umgebungsluft.

Die Erfindung lässt sich auch in einer Anwendung verwenden, bei dem kein Beatmungskreislauf realisiert wird.

Das vom Patienten P ausgeatmete Gasgemisch At gelangt durch eine Exspirationsleitung 33 hindurch in die Umgebung.

Eine nur schematisch gezeigte patientenseitige Koppeleinheit 21, beispielsweise ein Mundstück oder eine Atemmaske oder ein Tubus, verbindet den Patienten P mit dem Beatmungskreislauf 40. Ein patientennaher Schlauch verbindet die patientenseitige Koppeleinheit 21 mit dem Basisteil eines Y-Stücks 22. Ein Schenkel des Y-Stücks 22 ist mit einer Inspirationsleitung 32 für die Einatmung (Inspiration) verbunden, der andere Schenkel mit einer Exspirationsleitung 33 für die Ausatmung (Exspiration).

Eine Fluidfördereinheit 24a saugt Gas an und erzeugt einen permanenten Strom von atembarem Gasgemisch Gg durch die Inspirationsleitung 32 auf das Y-Stück 22 und auf die patientenseitigen Koppeleinheit 21 und damit auf den Patienten P zu. Der Beatmungskreislauf 40 wird von der Fluidfördereinheit 24a und optional von einem Atembeutel 26, der manuell betätigt werden kann, in Gang gehalten. Die Fluidfördereinheit 24a kann insbesondere als ein Gebläse, eine Pumpe oder eine Kolben-Zylinder-Einheit realisiert sein.

Ein Rückschlagventil (non-return valve) 23a lässt einen Gasfluss durch die Inspirationsleitung 32 auf das Y-Stück 22 zu durch und sperrt einen Gasfluss in die umgekehrte Richtung. Ein Rückschlagventil 23b lässt einen Gasfluss durch die Exspirationsleitung 33 vom Y-Stück 22 weg passieren und sperrt einen Gasfluss in die umgekehrte Richtung.

Im Ausführungsbeispiel fördert die Fluidfördereinheit 24a kontinuierlich das Gasgemisch Gg. Ein ansteuerbares Ventil 24b, bevorzugt ein Proportionalventil, lässt je nach seiner Stellung den Strom des Gasgemischs, den die Fluidfördereinheit 24a erzeugt hat, passieren oder sperrt ihn ganz oder vollständig, trägt dadurch zur Erzeugung der einzelnen Beatmungshübe (ventilation strokes) bei und legt die Amplituden und Frequenzen dieser Beatmungshübe fest.

Ein PEEP-Ventil 24c (PEEP = "positive end-expiratory pressure") stellt außerdem sicher, dass auch am Ende der Ausatmung oder bei einer kurzfristigen Öffnung oder Unterbrechung des Beatmungskreislaufs 40 ein ausreichend großer Luftdruck in der Lunge des Patienten P aufrecht erhalten bleibt. Dadurch wird die Gefahr verringert, dass die Lunge des Patienten P durch einen zu geringen Druck kollabiert.

Ein Überdruckventil (pressure relief valve) 29 vermag einen Überdruck im Beatmungskreislauf 40 abzubauen, indem es bewirkt, dass bei einem zu hohen Druck Gas aus dem Beatmungskreislauf 40 entweicht. Bevorzugt wird das abgegebene Gas in eine Fortleitung für Narkosegas abgegeben und gelangt in einen nicht gezeigten Aufbereiter.

Ein nur schematisch gezeigtes signalverarbeitendes Beatmungs-Steuergerät (control unit) 35 empfängt ein Signal eines optionalen Drucksensors 58, der den Luftdruck $P_{amb}$ in der Umgebung des Anästhesiegeräts 1 und damit des Beatmungskreislaufs 40 misst, und ein Signal eines optionalen Temperatursensors 39, der die Umgebungstemperatur $Temp_{amb}$ misst. Außerdem empfängt das Beatmungs-Steuergerät 35 Messwerte eines Drucksensors 36, der den aktuellen Druck im Beatmungskreislauf 40 misst, beispielsweise den am Patienten P anliegenden Beatmungsdruck (pressure in airway, $P_{aw}$), in einer Ausgestaltung als Differenzdruck relativ zum Umgebungsdruck $P_{amb}$. Die Messposition des Drucksensors 36 kann in der Inspirationsleitung 32 oder im oder wie gezeigt am oder nahe dem Y-Stück 22 sein. Bevorzugt befindet sich die Messposition des Drucksensors 36 an dem patientennahen Schlauch. Das Beatmungs-Steuergerät 35 steuert die Fluidfördereinheit 24a, das Ventil 24b, den Narkosemitteldosierer 31 und weitere Bestandteile des Beatmungskreislaufs 40 an, um eine gewünschte künstliche Beatmung und Narkotisierung des Patienten P zu realisieren.

Häufig wird gewünscht, dass das Gasgemisch Gg, welches dem Patienten P zugeführt wird, eine bestimmte vorgegebene Eigenschaft erfüllt. Insbesondere soll die Konzentration eines Bestandteils des Gasgemischs Gg in einem vorgegebenen Sollbereich liegen. Insbesondere soll oft der Anteil von reinem Sauerstoff ($O_2$) in einem vorgegebenen Sollbereich liegen, beispielsweise zwischen 40 Vol.-% und 50 Vol.-% oder auch zwischen 25 Vol.-% und 30 Vol.-%. Oder der Anteil von Narkosemittel soll in einem vorgegebenen Bereich liegen. Im Folgenden wird der Begriff "Konzentration" eines Gasbestandteils in einem Gasgemisch verwendet. Ein synonymer Begriff ist "Anteil".

Zu diesem Zweck wird dem Gasgemisch Gg, At im Beatmungskreislauf 40 eine Probe (im Folgenden: eine Gasprobe Gp) über eine Gasprobe-Fluidführungseinheit in Form eines Entnahmeschlauchs 52 entnommen (abgezweigt), analysiert und über einen Abführschlauch 56 wieder in den Beatmungskreislauf 40 eingespeist. Der Entnahmeschlauch 52 beginnt in einem Abzweigpunkt 34 zwischen der patientenseitigen Koppeleinheit 21 und dem Y-Stück 22. Weil der Abzweigpunkt 34 an dieser Stelle positioniert ist, enthält die Gasprobe Gp je nach dem Zeitpunkt des Abzweigens eine Probe aus dem atembaren Gasgemisch Gg oder aus dem ausgeatmeten Gasgemisch At. In den Entnahmeschlauch 52 gerät nämlich zeitweise ein Gasgemisch Gg aus der Inspirationsleitung 22 und zeitweise ein Gasgemisch At für die Exspirationsleitung 33. Am Abzweigpunkt 34 befindet sich optional ein nicht gezeigtes Ventil, welches in einer geschlossenen Stellung den Entnahmeschlauch 52 vom Beatmungskreislauf 40 abtrennt und welches sich vom Beatmungs-Steuergerät 35 ansteuern lässt. Bei vollständig geöffnetem oder fortgelassenem Ventil steht der Entnahmeschlauch 52 in einer nicht eingeschränkten Fluidverbindung mit dem Beatmungskreislauf 40. Der Abführschlauch 56 führt zu einem Einmündepunkt 37 flussaufwärts vom Kohlendioxidabsorber 25a.

Der Entnahmeschlauch 52 leitet die Gasprobe Gp zu einer Sensor-Anordnung 50. Diese Sensor-Anordnung 50 ist räumlich von der patientenseitigen Koppeleinheit 21 und von den Leitungen 32 und 33 entfernt und gehört bevorzugt ebenfalls zu dem schematisch gezeigten Beatmungsgerät 1. In Figur 1 ist die Sensor-Anordnung 50 zur Verdeutlichung außerhalb des Beatmungsgeräts 1 gezeigt.

Die Sensor-Anordnung 50 umfasst im Ausführungsbeispiel eine Pumpe 55, welche die Gasprobe Gp aus den Beatmungskreislauf 40 absaugt und durch den Entnahmeschlauch 52 hindurch ansaugt. Bevorzugt erzeugt die Pumpe 55 an der zum Entnahmeschlauch 52 hin zeigenden Seite dauerhaft einen Unterdruck und an der zum Abführschlauch 56 hin zeigenden Seite dauerhaft einen Überdruck. Die Pumpe 55 vermag einen Volumenfluss zu erzeugen, der bevorzugt zeitlich konstant ist und beispielsweise 200 ml/min beträgt. Am Eingang der Sensor-Anordnung 50 ist eine Wasserfalle 51 angeordnet, wobei in der Wasserfalle 51 Feuchtigkeit kondensiert und gesammelt wird. Dadurch wird der Gehalt von Wasser in der Gasprobe Gp reduziert.

Ein Sensor 54 vermag Signale zu erzeugen, wobei die Signale mit der jeweils gemessenen Konzentration von $CO_2$ und von $N_2O$ in der abgesaugten Gasprobe Gp korrelieren. Bevorzugt umfasst dieser Sensor 54 einen Infrarot-Messkopf, der das Dipol-Moment von Molekülen in der Gasprobe Gp ausnutzt und die Absorption von infrarot-aktiven Gasen quantitativ auswertet, um die jeweilige Konzentration zu bestimmen. Bevorzugt umfasst der Sensor 54 eine Strahlungsquelle, die elektromagnetische Strahlung emittiert, sowie einen Detektor, der die Intensität von auftreffender elektromagnetischer Strahlung misst und ein entsprechendes Signal erzeugt.

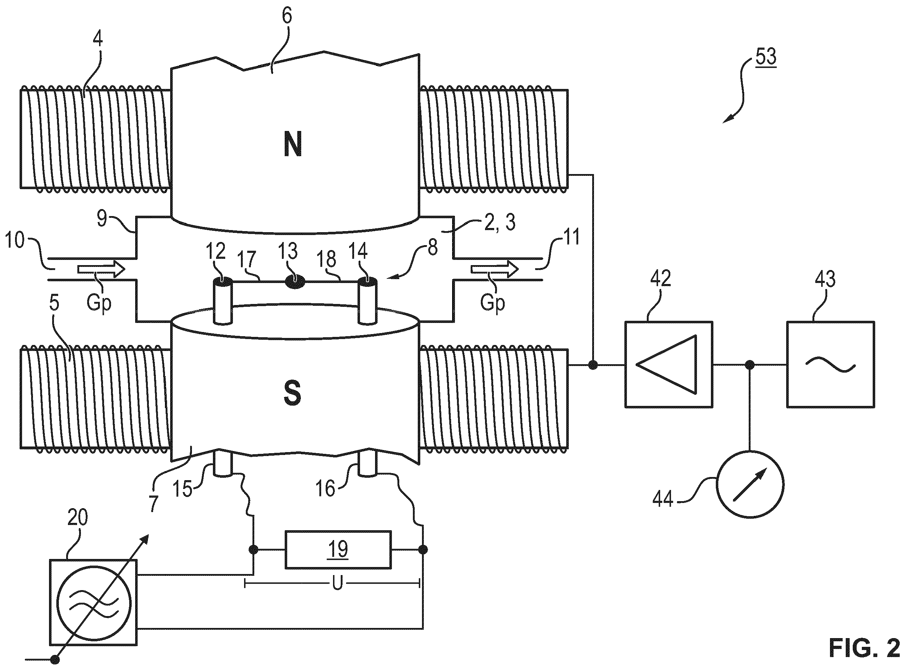

Ein Sensor 53 vermag ein Signal zu erzeugen, welches u.a. mit der Konzentration von $O_2$ oder eines sonstigen paramagnetischen Bestandteils der Gasprobe Gp korreliert. Der Sensor 53 wird weiter unten mit Bezug auf Figur 2 bis Figur 5 näher beschrieben.

Außerdem umfasst die Sensor-Anordnung 50 einen Drucksensor 57, der den Druck $P_{cell}$ der Gasprobe Gp am Eingang der Sensor-Anordnung 50 misst. Dieser Druck $P_{cell}$ ist zeitlich veränderlich, weil der Druck im Beatmungskreislauf 40 oszilliert und weil der Entnahmeschlauch 52 mit dem Beatmungskreislauf 40 in einer Fluidverbindung steht, wenn am Abzweigpunkt 34 kein Ventil vorhanden ist oder solange das optionale Ventil am Abzweigpunkt 34 geöffnet ist. Bei fehlendem oder geöffnetem Ventil pflanzt sich der Druck im Beatmungskreislauf 40 annähernd mit Schallgeschwindigkeit zur Sensor-Anordnung 50 fort.

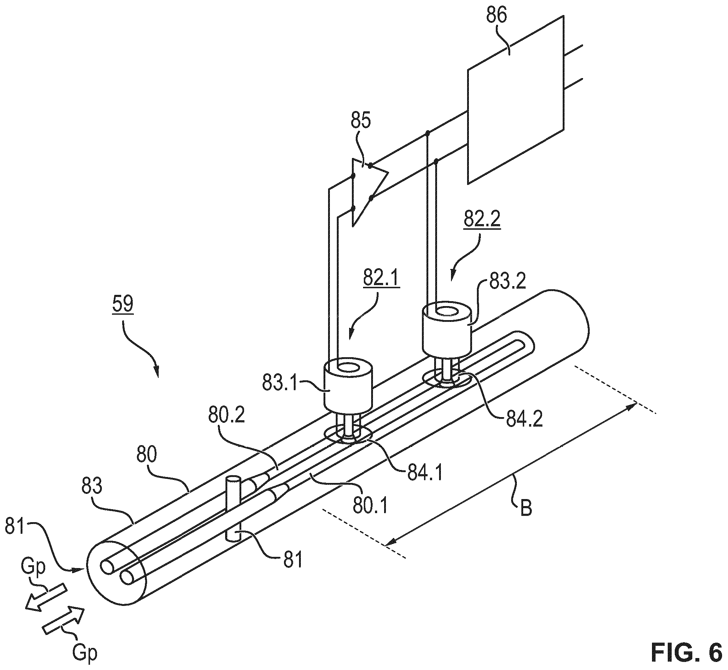

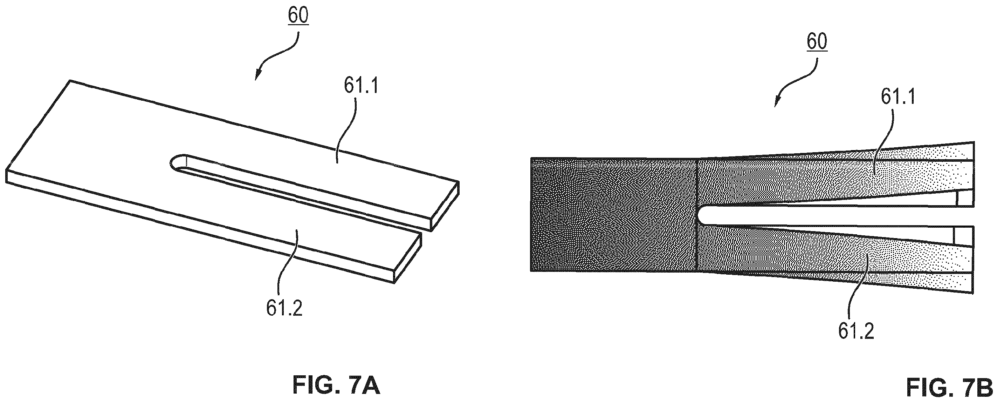

Ein Dichtesensor 59, 60 misst die Dichte der Gasprobe Gp, welche durch die Sensor-Anordnung 50 fließt. Dieser Dichtesensor 59, 60 wird weiter unten mit Bezug auf Figur 6 und Figur 7 beschrieben. Im Beispiel von Figur 1 ist der Dichtesensor 59, 60 parallel zu den Sensoren 53, 54, 57 angeordnet. Möglich ist auch, dass die Sensoren 53, 54, 57 in Reihe mit dem Dichtesensor 59, 60 angeordnet sind und die abgezweigte Gasprobe Gp durch alle vier Sensoren fließt.

Die Sensor-Anordnung 50, die Wasserfalle 51, die Signalverarbeitungseinheit 30 und die Schläuche 52 und 56 gehören zusammen zu einem Messsystem 100, welches die Gasprobe Gp aus dem Beatmungskreislauf 40 absaugt, untersucht und wieder in den Beatmungskreislauf 40 zurückspeist. Das Messsystem 100 gehört zur Beatmungs-Anordnung 200.

Figur 2 zeigt beispielhaft einen Sensor 53, der die jeweilige Konzentration von Sauerstoff ($O_2$) und einem weiteren Gas in der Gasprobe Gp zu messen vermag. Dieser Sensor 53 nutzt die Tatsache aus, dass Sauerstoff ein paramagnetisches Gas ist, während jedes weitere Gas, das in der Gasprobe Gp vorhanden ist oder vorhanden sein kann, nicht paramagnetisch ist.

Zwei Polschuhe 6, 7 und zwei Feldspulen 4, 5 erzeugen ein Magnetfeld. Zwischen den beiden Polschuhen 6, 7 tritt ein Luftspalt 3 auf, der als eine Messkammer 2 fungiert. Diese Messkammer 2 wird durch die beiden Polschuhe 6, 7 sowie durch eine Wandung 9 begrenzt. In die Wandung 9 sind ein Einlass 10 und ein Auslass 11 eingelassen.

Ein Thermoelement 8 ist in zwei Verbindungsstellen 12 und 14 an zwei Stützdrähten 15, 16 befestigt, wobei die beiden Stützdrähte 15, 16 durch den unteren Polschuh 7 hindurchgeführt sind und in einem thermischen Kontakt mit dem unteren Polschuh 7 stehen. Das Thermoelement 8 umfasst zwei Drähte 17, 18, die an einer Verbindungsstelle 13 miteinander verbunden sind. Eine Spannungsquelle 20 legt eine Wechselspannung an die beiden Stützdrähte 15, 16 und damit an das Thermoelement 8 an. Dadurch wird das Thermoelement 8 auf eine Arbeitstemperatur erhitzt, die größer ist als die Temperatur der Gasprobe Gp in der Messkammer 2. Eine elektrische Spannung U, die an einem Messwiderstand 19 anliegt, wird gemessen. Diese elektrische Spannung U enthält einen Wechselspanungs-Anteil und einen Gleichspannungs-Anteil.

Die aktuelle Arbeitstemperatur des Thermoelements 8 wird an der Verbindungsstelle 13 gemessen. Diese Temperatur hängt einerseits von der Spannung U ab, die zwischen den Stützdrähten 15, 16 auftritt, und andererseits von der Wärmeleitfähigkeit λ der Gasprobe Gp in der Messkammer 2. Eine Regelung (closed-loop control) wird mit dem Ziel durchgeführt, dass die Arbeitstemperatur des Thermoelements 8 auf einen konstanten Wert geregelt wird. Die Stellgröße ist die zeitlich veränderliche Spannung U, welche die Spannungsquelle 20 an die Stützdrähte 15, 16 anlegt. Weil die Temperatur des Thermoelements 8 idealerweise gleichbleibt, korreliert eine elektrische Detektionsgröße des Thermoelements 8 mit der Wärmeleitfähigkeit λ der Gasprobe Gp in der Messkammer 2.

Eine Realisierungsform, um die Detektionsgröße zu messen, ist die folgende: Als Detektionsgröße wird die zeitlich veränderliche elektrische Leistung, die dem Thermoelement 8 zugeführt wird, gemessen. Die beiden Stützdrähte 15 und 16 sind durch den elektrischen Messwiderstand 19 elektrisch miteinander verbunden. Die elektrische Spannung U, die an diesem Messwiderstand 19 abfällt, wird gemessen. Außerdem wird die Stärke des elektrischen Stroms, der durch das Thermoelement 8 fließt, gemessen. Die elektrische Leistung hängt bekanntlich von der Spannung und der Stromstärke ab. Möglich ist auch, die elektrische Spannung U als Detektionsgröße zu verwenden.

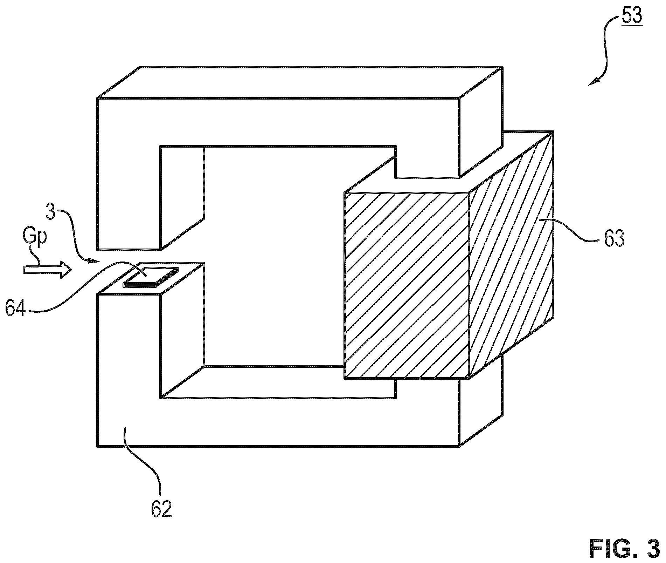

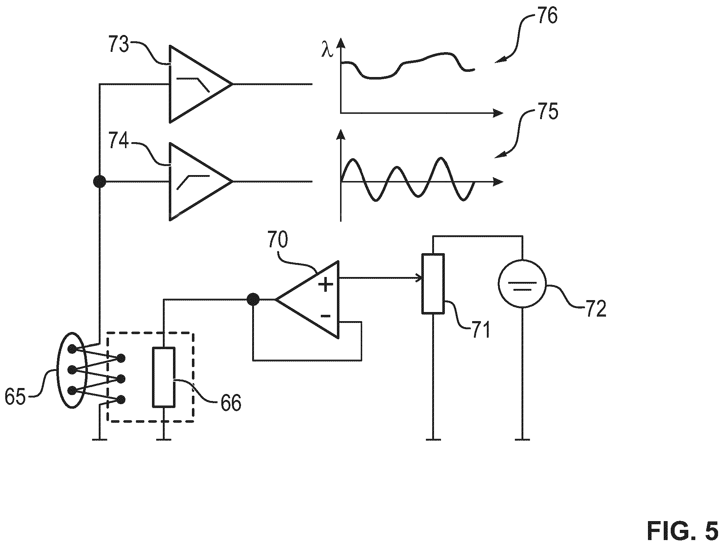

Eine Spannungsquelle 43 ist über einen Leistungsverstärker 42 mit der einen Feldspule 5 verbunden, und die andere Feldspule 4 ist mit elektrischer Masse verbunden. Die Spannungsquelle 43 gibt eine sinusförmige elektrische Spannung ab. Diese Spannung erzeugt ein sinusförmig variierendes Magnetfeld in der Messkammer 2. Das Thermoelement 8, das auf eine gleichbleibende Temperatur erhitzt wird, gibt an die Gasprobe Gp in der Messkammer 2 eine Wärmemenge pro Zeiteinheit ab. Diese abgegebene Wärmemenge pro Zeiteinheit korreliert mit der gemessenen elektrischen Leistung, die dem Thermoelement 8 zugeführt wird. Weil die Feldstärke des Magnetfelds in der Messkammer 2 periodisch oszilliert, oszilliert auch die abgegebene Wärmemenge pro Zeiteinheit - vorausgesetzt als Teil der Gasprobe Gp befindet sich ein paramagnetisches Gas in der Messkammer 2. Die Detektionsgröße korreliert mit der Wärmeleitfähigkeit $\lambda$ der Gasprobe Gp. Unter Verwendung der Detektionsgröße werden ein abhängig von der Oszillation des Magnetfelds oszillierendes Signal, beispielsweise eine Wechselspannung, und ein nicht oder weniger oszillierendes Signal, beispielsweise eine Gleichspannung, generiert. Das oszillierende Signal korreliert mit der Wärmeleitfähigkeit des paramagnetischen Teils der Gasprobe Gp und damit mit der $O_2$-Konzentration. Diese Wärmeleitfähigkeit wird auch als magnetisch modulierte Wärmeleitfähigkeit $\lambda_{2f}$ bezeichnet. Das andere Signal korreliert mit der (gesamten) Wärmeleitfähigkeit $\lambda$ der gesamten Gasprobe Gp. Figur 3, Figur 4 und Figur 5 zeigen eine weitere Ausgestaltung des Sensors 53. Gleiche Bezugszeichen haben die gleiche Bedeutung wie in Figur 2. Die Schaltung von Figur 5 lässt sich entsprechend auch für die Ausgestaltung gemäß Figur 2 anwenden.

Ein Elektromagnet 62 mit einer elektrischen Spule 63 erzeugt im Luftspalt 3 ein zeitlich veränderliches, bevorzugt oszillierendes, Magnetfeld, vgl. Figur 3. Die zu untersuchende Gasprobe Gp erreicht diesen Luftspalt 3. Der zeitliche Verlauf der Stärke des erzeugten Magnetfelds wird durch die Ansteuerung der Spule 63 festgelegt. Bevorzugt hat die Stärke des Magnetfelds einen sinusförmigen Verlauf. Im Luftspalt 3 ist eine Messeinheit 64 angeordnet, die als Chip implementiert ist, bevorzugt als Halbleiter-Chip, der beispielsweise unter Verwendung von dotiertem Silizium hergestellt ist.

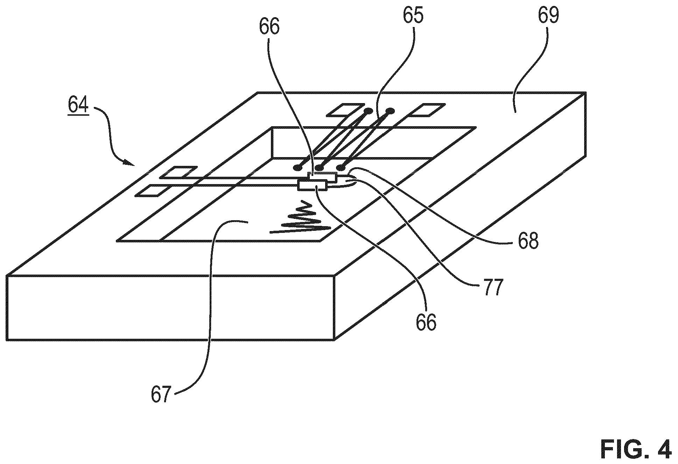

Figur 4 zeigt die Messeinheit 64 im Detail. Die Messeinheit 64 umfasst ein elektrisch steuerbares Wärmeleitungs-Messelement 65, eine elektrisch steuerbare Heizeinheit 66 und eine Membrane 67 in einem Rahmen 69. Eine Gasprobe Gp kann durch Löcher in der Membrane 67 oder um die Membrane 67 herum zu den Elementen 65, 66 gelangen. Die Heizeinheit 66 kann eine elektrisch leitfähige Widerstandsstruktur sein, die auf der Membrane 67 abgeschieden ist, oder als Heizdraht ausgestaltet sein. Gezeigt werden zwei Heizelemente der Heizeinheit 66, die durch ein Verbindungselement 68 elektrisch miteinander verbunden sind. Das Wärmeleitungs-Messelement 65 und die Heizeinheit 66 können auch als ein einstückiges Element ausgestaltet sein, welches einen temperaturabhängigen elektrischen Widerstand aufweist.

Die Heizeinheit 66 heizt das Messelement 65 auf eine Arbeitstemperatur auf, die größer als die Temperatur einer Gasprobe Gp in der Messkammer 2 ist. Das beheizte Messelement 65 misst die Temperatur an der Messposition 77. In der gezeigten Realisierungsform misst das Messelement 65 eine Thermospannung und nutzt den Seebeck-Effekt aus. Die Wärmeleitfähigkeit der Gasprobe Gp an der Messposition 77 verändert sich synchron zu dem zeitlich variierenden Magnetfeld, welches der Elektromagnet 62 erzeugt - vorausgesetzt, die Gasprobe Gp an der Messposition 77 enthält mit ausreichend großer Konzentration ein paramagnetisches Gas. Eine höhere Wärmeleitfähigkeit führt dazu, dass die Wärmeenergie besser weggeleitet wird. Dies wiederum bewirkt eine niedrigere Temperatur, welche ihrerseits eine niedrigere Thermospannung bewirkt.

Figur 5 zeigt beispielhaft eine Auswerteschaltung mit folgenden Bestandteilen:

- einem Verstärker 70, der als Impedanzwandler geschaltet ist,
- einem Spannungsteiler 71 mit variablem Abgriff,
- eine Gleichspannungsquelle 72,
- einem Tiefpass-Filter 73 und
- einen Hochpass-Filter 74, der parallel zum Tiefpass-Filter 73 geschaltet ist.

Die Heizeinheit 66 ist über den Verstärker 70 und den Spannungsteiler 71 mit der Gleichspannungsquelle 72 verbunden. Das Ausgangssignal des Wärmeleitungs-Messelements 65 wird über den Tiefpass-Filter 73 sowie über den Hochpass-Filter 74 geführt. Am Ausgang des Hochpass-Filters 74 liegt ein Signal 75 an, welches abhängig von der Oszillation des Magnetfelds oszilliert. Das Signal 75 korreliert mit der magnetisch modulierten Wärmeleitfähigkeit $\lambda_{2f}$ und damit mit der Konzentration des paramagnetischen Gases, beispielsweise mit der Konzentration von Sauerstoff, in der Gasprobe Gp. Am Ausgang des Tiefpass-Filters 73 liegt ein Signal 76 an, welches überhaupt nicht oszilliert oder zumindest nicht synchron mit der Magnetfeld-Oszillation. Das Signal 76 korreliert mit der Wärmeleitfähigkeit $\lambda$ der gesamten Gasprobe Gp.

Weil die Magnetfeldstärke oszilliert, umfasst die zugeführte Wärmemenge pro Zeiteinheit eine Überlagerung eines zeitlich gleichbleibenden Anteils und eines periodischen schwankenden Anteils. Der periodisch schwankende Anteil korreliert mit der Wärmeleitfähigkeit und damit mit der Konzentration von Sauerstoff in der Messkammer 2. Der gleichbleibende Anteil, also der Anteil, der nicht abhängig von der Stärke des Magnetfelds oszilliert, korreliert mit der Wärmeleitfähigkeit der gesamten Gasprobe Gp in der Messkammer 2. Zu dieser Wärmeleitfähigkeit $\lambda$ der Gasprobe Gp tragen die Wärmeleitfähigkeit des Sauerstoffs und die jeweilige Wärmeleitfähigkeit jedes anderen Bestandteils der Gasprobe Gp bei. Beide Anteile werden gemessen. Der Sensor 53 liefert also zwei Signale:

- ein abhängig von der Magnetfeldstärke oszillierendes, hier periodisch schwankendes Signal, welches ein Maß für die Konzentration von Sauerstoff oder einem sonstigen paramagnetischen Gas in der Messkammer 2 ist (periodisch schwankender Anteil, Wechselspannung), und

- ein Signal für die Wärmeleitfähigkeit $\lambda$ der Gasprobe Gp in der Messkammer 2, wobei dieses Signal nicht oder wenigstens nicht abhängig von der Magnetfeldstärke oszilliert (gleichbleibender Anteil, Gleichspannung).

Optional werden ein gemessener Wert für die Konzentration eines Bestandteils und / oder für die Wärmeleitfähigkeit $\lambda$ auf einer Anzeigevorrichtung 44 angezeigt, vgl. Figur 2.

Erfindungsgemäß wird außerdem die Dichte der Gasprobe Gp gemessen. Figur 6 zeigt schematisch eine Ausführungsform eines Dichtesensors 59 in Form eines mechanischen Biegeschwingers, Figur 7 eine Ausführungsform eines elektronischen Biegeschwingers 60, der als ein Schwingquarz realisiert ist. In einer Ausgestaltung ist der Dichtesensor 59, 60 parallel zu dem Sensor 53 geschaltet, der die Wärmeleitfähigkeit und die magnetisch modulierte Wärmeleitfähigkeit misst und weiter oben mit Bezug auf Figur 2 bis Figur 5 beschrieben wurde. Möglich ist, dass der Sensor 53 als ein Chip realisiert ist und der Dichtesensor 60 auf diesem Chip montiert ist und von diesem Chip mit elektrischer Spannung versorgt wird.

Das nachfolgend beschriebene Prinzip der Dichtemessung ist seit langem bekannt, aber nicht für die hier beschriebene Anwendung. Im Ausführungsbeispiel misst der Dichtesensor 59, 60 die Dichte $\rho$ der Gasprobe Gp, die durch die Sensor-Anordnung 50 fließt.

Zunächst wird ein mechanischer Biegeschwinger 59 mit Bezug auf Figur 6 beschrieben. Die Gasprobe Gp, deren Dichte $\rho$ gemessen werden soll, wird durch ein U-förmiges Rohr 80 mit zwei Schenkeln 80.1, 80.2 geleitet. Das Rohr 80 befindet sich in einem Gehäuse 83 und ist aus einem Material hergestellt, welches den Bestandteilen der Gasprobe Gp chemisch standhalten kann, beispielsweise aus einem Glas oder einem Stahl. Die beiden Schenkel 80.1, 80.2 des Rohrs 80 bilden die Federelemente eines Biegeschwingers. Dieses Prinzip ähnelt dem Prinzip einer Stimmgabel. Die beiden Schenkel 80.1, 80.2 sind an einer Befestigungsstelle 81 eingespannt. Ein Bereich B des Rohrs 80 kann in Schwingungen versetzt werden. Dieser Bereich B hat ebenfalls die Form eines U, umfasst jeweils ein Segment jedes Schenkels 80.1, 80.2 und wird von der Befestigungsstelle 81 begrenzt.

In einer Realisierungsform lässt sich das Rohr 80 zum Zwecke einer Messung zeitweise verschließen. In einer anderen Realisierungsform durchströmt auch während der Messung die Gasprobe Gp das Rohr 80.

Ein schematisch gezeigtes Stellglied regt diesen Biegeschwinger zu ungedämpften Schwingungen an. In der gezeigten Realisierungsform wird das Stellglied durch zwei Umwandler 82.1, 82.2 realisiert. Jeder Umwandler 82.1, 82.2 umfasst jeweils eine Spule 83.1, 83.2 und einen Magneten 84.1, 84.2. In einem Modus wird an jeden Umwandler 82.1, 82.2 eine elektrische Wechselspannung angelegt, der Umwandler 82.1, 82.2 erzeugt mechanische Schwingungen, und diese mechanischen Schwingungen werden auf das Rohr 80 übertragen, und zwar bevorzugt durch eine mechanische Verbindung zwischen dem Magneten 84.1, 84.2 und dem Rohr 80.

Die beiden Schenkel 80.1, 80.2 spannen eine Ebene auf. Die beiden Richtungen der durch die Anregung erzeugten Schwingungen stehen orthogonal auf dieser Ebene. Ein Bereich B des Rohrs 80 wird in Schwingungen versetzt. Anschließend wird das Stellglied 84 ausgeschaltet. Danach schwingt der Bereich B weiter, und zwar nach einer Einschwingphase mit seiner Eigenfrequenz.

An der Schwingung nehmen der Bereich B des Rohrs 80 sowie derjenige Teil der Gasprobe Gp, der sich im Inneren dieses Bereichs B befindet, teil. Dieses schwingende System weist eine Masse auf, wobei diese Masse die Summe der Masse des Bereichs B und der Masse des Gasproben-Teils im Bereich B ist. Die Masse des Bereichs B ist durch die Konstruktion vorgegeben und bleibt konstant. Das Volumen, das der Bereich B für die Gasprobe Gp bereitstellt, bleibt ebenfalls konstant. In der Regel kann die Masse desjenigen Teils der Gasprobe Gp, der sich im Bereich B befindet, während einer Messung als konstant angesehen werden. Die gesuchte Dichte $\rho$ ist dann proportional zur Masse des Gasproben-Teils.

Anschließend werden die beiden Umwandler 82.1, 82.2 abgeschaltet. Nunmehr schwingt das gerade beschriebene System mit einer Eigenfrequenz $\tau$. Diese Eigenfrequenz $\tau$ hängt von der Masse des Systems ab und wird gemessen. In der gezeigten Realisierungsformen fungieren die beiden Umwandler 82.1, 82.2 nach dem Abschalten in einem anderen Modus und wandeln die mechanischen Schwingungen des Bereichs B in eine Wechselspannung um. Die Frequenz dieser Wechselspannung ist gleich der Eigenfrequenz $\tau$ des Bereichs B. Ein Verstärker 85 verstärkt diese Wechselspannung. Eine Auswerteeinheit 86 empfängt ein Signal des Verstärkers 85 und liefert die gesuchte Dichte $\rho$.

Die gesuchte Dichte $\rho$ hängt wie folgt mit der gemessenen Eigenfrequenz $\tau$ zusammen:

$$(1) \qquad \rho = A * \tau^2 - B.$$

A und B sind zwei Konstanten des Dichtesensors (Biegeschwingers) 59. In einer vorhergehenden Kalibrierung werden diese beiden Konstanten A und B empirisch ermittelt, beispielsweise mittels einer Regressionsanalyse. Hierfür werden mindestens zwei verschiedene Gase mit bekannten und sich voneinander unterscheidenden Dichten durch den Dichtesensor 59 geschickt, und die jeweilige resultierende Eigenfrequenz wird gemessen.

Figur 7 zeigt schematisch einen Biegeschwinger 60 in Form eines Schwingquarzes. Dieser Schwingquarz hat die Form einer Stimmgabel mit zwei Schenkel 61.1 und 61.2. Figur 7A zeigt schematisch diesen Schwingquarz. An diesen Biegeschwinger 60 werden Elektroden dergestalt angeschlossen, dass zwei entgegengesetzte Felder in x-Richtung auftreten. Hierdurch wird eine Biegung in z-Richtung erzwungen. Figur 7B veranschaulicht die Schwingungen dieser beiden Schenkel 61.1 und 61.2, wobei die x-Richtung und die z-Richtung in der Zeichenebene liegen. Durch eine Rückkopplung wird bewirkt, dass die erzwungene Frequenz gleich der Eigenfrequenz $\tau$ ist.

Ein solcher Biegeschwinger 60 wird beispielsweise in Th. Voglhuber-Brunnmeier et al.: Fluid Sensing Using Quartz Tuning Forks - Measurement Technology and Applications, Sensors, Vol. 19 (2019) No. 10, beschrieben.

In einer Ausgestaltung wird der folgende Zusammenhang verwendet:

$$(2) \qquad \rho = \left[ -\frac{B}{2A} + \sqrt{\left(\frac{B}{2A}\right)^2 + \left(\frac{\tau - C}{A}\right)^2} \right]^2$$

Wiederum sind A, B und C gerätespezifische Konstanten, die vorab durch Kalibrierung ermittelt werden. Weil drei Konstanten zu ermitteln sind, werden zur Kalibrierung mindestens drei verschiedene Gase mit bekannten und sich voneinander unterscheidenden Dichten verwendet.

Wie bereits dargelegt, soll die Sensor-Anordnung 50 die jeweilige Konzentration (Anteil) in Vol.-% von Sauerstoff und von Narkosemittel (Anästhesiegas) in der abgezweigten Gasprobe Gp messen. In dieser Gasprobe Gp können weitere Bestandteile vorhanden sein, welche ohne eine geeignete Gegenmaßnahme diese Messung verfälschen können. Insbesondere kann das Edelgas Argon (Ar) in der Gasprobe Gp die Messung verfälschen. Bekanntlich beträgt der Anteil von Argon in der Umgebungsluft etwa 0,93 Vol.-%. Die Konzentration von Argon in der Gasprobe Gp kann aber deutlich höher sein als die von Argon in der Umgebungsluft. Diese Situation tritt insbesondere dann auf, wenn die Konzentration von Sauerstoff im Beatmungskreislauf 40 und daher in der Gasprobe Gp größer als die Konzentration von Sauerstoff in der Umgebungsluft ist, und zwar insbesondere dann, wenn das Gasgemisch Gg aus Umgebungsluft erzeugt und die Sauerstoff-Konzentration mit Hilfe eines sogenannten Sauerstoff-Konzentrators vergrößert worden ist.

In manchen Situationen wird eine Information über die gemessene Argon-Konzentration zusätzlich von einem weiteren Sensor oder einem sonstigen Bestandteil des Anästhesiegeräts 1 verwendet. Beispielsweise wird die gemessene Argon-Konzentration mit einer oberen Schranke verglichen. Sobald diese obere Schranke für die Argon-Konzentration erreicht ist, wird der Beatmungskreislauf 40 gespült.

Im Ausführungsbeispiel enthält die Gasprobe Gp n verschiedene Bestandteile, darunter Stickstoff ($N_2$), reinen Sauerstoff ($O_2$), Kohlendioxid ($CO_2$), ein Anästhesiegas (A-Gas), Lachgas ($N_2O$), Wasser ($H_2O$) und Argon (Ar). Die jeweilige Dichte $\rho(x_i)$ (i=1, ..., n) und die jeweilige Wärmeleitfähigkeit $\lambda(x_i)$ dieser n Bestandteile sind bekannt und werden vorgegeben. Bevorzugt hat die Sensor-Anordnung 50 wenigstens zeitweise Lesezugriff auf einen Datenspeicher, in dem die Dichten und die Wärmeleitfähigkeiten abgespeichert sind. Die nachfolgende Tabelle zeigt beispielhaft die jeweilige Dichte und die jeweilige Wärmeleitfähigkeit von Bestandteilen, die im Beatmungskreislauf 40 und damit in der Gasprobe Gp vorkommen oder vorkommen können, wobei die Dichten und die Wärmeleitfähigkeiten sich auf eine Referenz-Umgebungstemperatur von 15 Grad C und einen Referenz-Umgebungsdruck von 1 bar beziehen.

*Tabelle 1 Wärmeleitfähigkeit und Dichte von verschiedenen Gasbestandteilen*

| Gas $x_i$ | Dichte $\rho(x_i)$ [gdm³] | Wärmeleitfähigkeit $\lambda(x_i)$ [WmK] |
|---|---|---|
| Sauerstoff ($O_2$) | 1,336 | 0,02615 |
| Stickstoff ($N_2$) | 1,169 | 0,02566 |
| Kohlendioxid ($CO_2$) | 1,8474 | 0,01643 |
| trockene Umgebungsluft | 1,209 | 0,02603 |
| Wasserdampf | 1 | 0,0199 |
| Isofluran | 7,701 | 0,00942 |

(fortgesetzt)

| Gas $x_i$ | Dichte $\rho(x_i)$ [gdm³] | Wärmeleitfähigkeit $\lambda(x_i)$ [WmK] |
|---|---|---|
| Lachgas (N₂O) | 1,848 | 0,0173 |
| Argon (Ar) | 1,669 | 0,01782 |

Die Gasbestandteile haben unterschiedliche Dichten. Man sieht, dass das beispielhaft genannte Narkosemittel Isofluran eine um ein Mehrfaches größere Dichte aufweist als die übrigen Gasbestandteile.

Wie bereits dargelegt, misst ein optionaler Drucksensor 36 den Druck im Beatmungskreislauf 40 und liefert bevorzugt den Atemwegsdruck $P_{AW}$. Der optionale Drucksensor 58 misst den Druck $P_{amb}$ in der Umgebung, der optionale Temperatursensor 39 die Umgebungstemperatur $Temp_{amb}$, und ein optionaler Drucksensor 57 den Druck $P_{cell}$ der Gasprobe Gp am Eingang der Sensor-Anordnung 50. Die in Tabelle 1 aufgelisteten Werte beziehen sich auf eine bestimmte Referenz-Umgebungstemperatur und einen bestimmten Referenz-Umgebungsdruck. Optional verwendet die Signalverarbeitungseinheit 30 Signale von den Sensoren 36, 58 und / oder 39, um die tatsächliche Wärmeleitfähigkeit und die tatsächliche Dichte der möglichen Gasbestandteile bei der tatsächlichen Umgebungstemperatur $Temp_{amb}$ und dem tatsächlichen Umgebungsdruck $P_{amb}$ herzuleiten.

Gesucht ist die jeweilige Konzentration $\xi(x_i)$, also der Anteil in Vol.-%, jedes Bestandteils $x_i$ (i=1, ..., n).

In vielen Fällen gilt mit ausreichender Genauigkeit, dass die Dichte $\rho$ der Gasprobe Gp ein gewichtetes Mittel der jeweiligen Dichten $\rho(x_i)$ der n Bestandteile ist, dass also gilt:

$$(3) \qquad \rho = \xi(x_1)^*\rho(x_1) + \ldots + \xi(x_n)^*\rho(x_n).$$

Die gesuchten Konzentrationen $\xi(x_1)$, ..., $\xi(x_n)$ der Gasbestandteile $x_1$, ..., $x_n$ fungieren als die Gewichtsfaktoren.

Weiter oben wurde mit Bezug auf Figur 2 bis Figur 5 ein Sensor 53, der eine Detektionsgröße misst, die mit der Wärmeleitfähigkeit $\lambda$ der Gasprobe Gp korreliert. Im Beispiel von Figur 2 ist dies eine Detektionsgröße des Thermoelements 8, im Beispiel von Figur 5 das Signal 76. In vielen Fällen gilt mit ausreichender Genauigkeit, dass die Wärmeleitfähigkeit $\lambda$ der Gasprobe Gp ein gewichtetes Mittel der jeweiligen Wärmeleitfähigkeiten $\lambda(x_i)$ der Bestandteile ist:

$$(4) \qquad \lambda = \xi(x_1)^*\lambda(x_1) + \ldots + \xi(x_n)^*\lambda(x_n).$$

Die gesuchten Konzentrationen $\xi(x_1)$, ..., $\xi(x_n)$ der Gasbestandteile $x_1$, ..., $x_n$ fungieren als die Gewichtsfaktoren.

Möglich ist auch, folgende funktionale Abhängigkeit zu verwenden:

$$(5) \qquad \lambda = \sum_{i=1}^{n} \frac{\lambda(x_i)}{1 + \sum_{\substack{k=1 \\ k \neq i}}^{n} G(i,k) \frac{\xi(x_k)}{\xi(x_i)}}$$

Die Faktoren G(i,k) (i=1, ..., n; k=1, ..., n) lassen sich vorab empirisch bestimmen.

Der Sensor 53 vermag außerdem die magnetisch modulierte Wärmeleitfähigkeit $\lambda_{2f}$ der Gasprobe Gp zu messen, beispielsweise mit Hilfe des periodischen Anteils der Detektionsgröße der Ausgestaltung gemäß Figur 2 oder mithilfe des oszillierenden Signals 75 der Schaltung von Figur 5. Weil $O_2$ ein paramagnetisches Gas ist, hängt $\lambda_{2f}$ wesentlich von der $O_2$-Konzentration $\xi(O_2)$ ab. Allgemein lässt sich folgende funktionale Abhängigkeit f anwenden:

$$(6) \qquad \lambda_{2f} = f[\xi(O_2), \xi(N_2O), \xi(CO_2), \xi(H_2O), \xi(Ar), Temp_{amb}, P_{cell}]$$

Die funktionale Abhängigkeit f ist vorgegeben. $Temp_{amb}$ bezeichnet die Umgebungstemperatur, die beispielsweise durch den Temperatursensor 39 gemessen wird. In der Regel unterscheidet sich die Temperatur der Gasprobe Gp nicht wesentlich von der Umgebungstemperatur $Temp_{amb}$. Die $O_2$-Konzentration geht annähernd linear ein. Der Druck $P_{cell}$ wird beispielsweise durch den Drucksensor 57 gemessen.

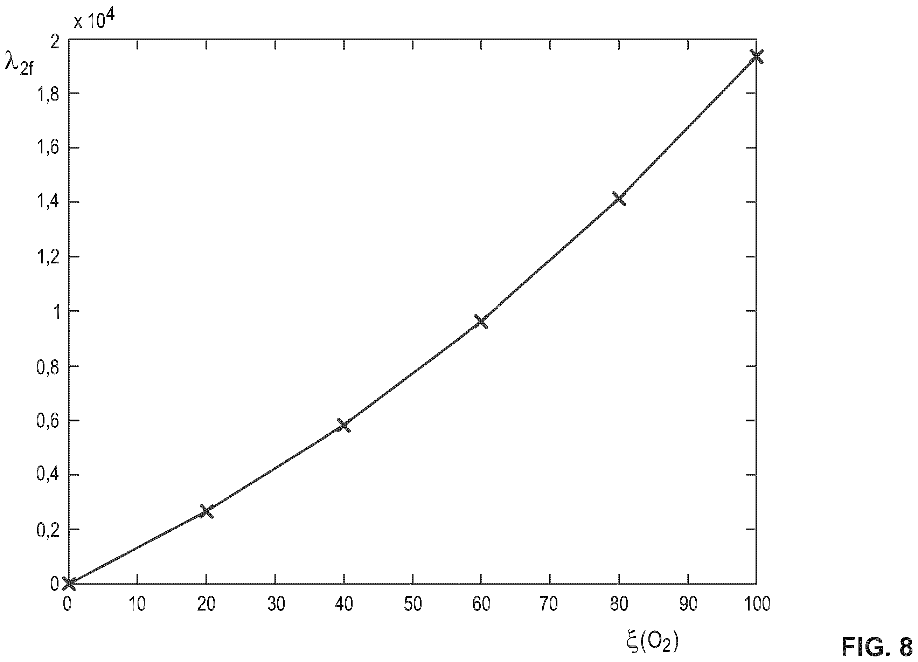

Figur 8 zeigt beispielhaft die magnetisch modulierte Wärmeleitfähigkeit $\lambda_{2f}$ eines Gasgemischs als Funktion der

$O_2$-Konzentration $\xi(O_2)$. In diesem Fall besteht das Gasgemisch lediglich aus Sauerstoff und $N_2O$. Auf der x-Achse ist die Konzentration $\xi(O_2)$ von Sauerstoff im Gasgemisch in Vol.-% aufgetragen, auf der y-Achse die magnetisch modulierte Wärmeleitfähigkeit $\lambda_{2f}$. Man erkennt, dass der Zusammenhang $\lambda_{2f} = \lambda_{2f}[\xi(O_2)]$ annähernd linear ist, aber nicht ideal linear.

Der Sensor 54 misst die $CO_2$-Konzentration $\xi(CO_2)$ und die $N_2O$-Konzentration $\xi(N_2O)$. Der Drucksensor 57 misst den Druck $P_{cell}$. Ein nicht gezeigter kapazitiver Sensor misst die $H_2O$-Konzentration $\xi(H_2O)$, also die Konzentration von Wasserdampf in der Gasprobe Gp. Der Dichtesensor 59, 60, der oben mit Bezug auf Figur 6 und Figur 7 beschrieben wurde, misst die Dichte $\rho$ der Gasprobe Gp.

Die $CO_2$-Konzentration $\xi(CO_2)$, die $N_2O$-Konzentration $\xi(N_2O)$ und die Wasserdampf-Konzentration $\xi(H_2O)$ werden direkt gemessen. Somit verbleiben drei unbekannte Anteile, nämlich die $O_2$-Konzentration $\xi(O_2)$, die Narkose-mittel-Konzentration $\xi(A\text{-}Gas)$ und die Argon-Konzentration $\xi(Ar)$. Um diese drei Unbekannten zu ermitteln, werden die drei Gleichungen (3), (4) und (6) verwendet. Zusammengenommen wird ein Gleichungssystem mit drei Unbekannten, nämlich $\xi(O_2)$, $\xi(A\text{-}Gas)$ und $\xi(Ar)$, und drei Gleichungen, nämlich (3), (4) und (6), verwendet.

In einer Realisierungsform werden für die drei Unbekannten $\xi(O_2)$, $\xi(A\text{-}Gas)$ und $\xi(Ar)$ drei Werte $\xi(x_1)$, $\xi(x_2)$ und $\xi(x_3)$ dergestalt berechnet, dass eine Zielfunktion Z mit

$$Z[\xi(x1),\xi(x2),\xi(x3)] =$$

$$(7) \qquad \alpha_1 \cdot \{\rho_{meas} - \rho[\xi(x_1),\xi(x_2),\xi(x3)]\}^2 +$$

$$\alpha_2 \cdot \{\lambda_{meas} - \lambda[\xi(x_1),\xi(x_2),\xi(x_3)]\}^2 +$$

$$\alpha_3 \cdot \{\lambda_{2f,meas} - \lambda_{2f}[\xi(x_1),\xi(x_2),\xi(x_3)]\}^2$$

minimiert wird. Hierbei bezeichnen $\rho_{meas}$ die gemessene Dichte, $\lambda_{meas}$ die gemessene Wärmeleitfähigkeit und $\lambda_{2f,meas}$ die gemessene magnetisch modulierte Wärmeleitfähigkeit. $\rho[\xi(x_1),\xi(x_2),\xi(x3)]$ bezeichnet die Dichte, die gemäß der Gleichung (3) auftritt, $\lambda[\xi(x_1),\xi(x_2),\xi(x_3)]$ die Wärmeleitfähigkeit gemäß der Gleichung (4) oder (5) und $\lambda_{2f}[\xi(x_1),\xi(x_2),\xi(x_3)]$ die magnetisch modulierte Wärmeleitfähigkeit gemäß der Gleichung (6). Die drei Faktoren $\alpha_1$, $\alpha_2$, $\alpha_3$ sind so gewählt, dass alle drei Summanden der Zielfunktion Z die gleiche Größenordnung aufweisen und / oder unterschiedliche Genauigkeitsanforderungen an die Messung der drei Konzentrationen $\xi(x_1),\xi(x_2),\xi(x_3)$ erfüllt werden. Die Summe der drei Faktoren $\alpha_1$, $\alpha_2$, $\alpha_3$ ergibt 1.

Offensichtlich ergibt die Summe der Gasbestandteil-Anteile 1, es gilt also

$$(8) \qquad \xi(x_1) + \ldots + \xi(x_n) = 1.$$

Mithilfe der Gleichung (8) lässt sich die $N_2$-Konzentration $\xi(N_2)$ herleiten.

**Bezugszeichenliste**

| 1 | Anästhesiegerät, gehört zur Beatmungs-Anordnung 200 |
|---|---|
| 2 | Messkammer des Sensors 53, im Luftspalt 3 gebildet |
| 3 | Luftspalt, stellt die Messkammer 2 bereit |
| 4, 5 | Feldspulen, erzeugen zusammen mit den Polschuhen 6, 7 ein Magnetfeld in der Messkammer 2 |
| 6, 7 | Polschuhe, erzeugen zusammen mit den Feldspulen 4, 5 ein Magnetfeld in der Messkammer 2 |
| 8 | Thermoelement, umfasst die Drähte 17, 18 und die Stützdrähte 15, 16 |
| 9 | Wandung der Messkammer 2 |
| 10 | Einlass in die Messkammer 2 |
| 11 | Auslass aus der Messkammer 2 |
| 12 | Verbindungsstelle zwischen dem Stützdraht 15 und dem Draht 17 |
| 13 | Verbindungsstelle zwischen den Drähten 17 und 18 |

| | |
|---|---|
| 14 | Verbindungsstelle zwischen dem Stützdraht 16 und dem Draht 18 |
| 15, 16 | Stützdrähte für das Thermoelement 8 |
| 17, 18 | Drähte des Thermoelements 8, sind in der Verbindungsstelle 13 miteinander verbunden |
| 19 | Messwiderstand des Thermoelements 8 |
| 20 | Spannungsquelle |
| 21 | patientenseitige Koppeleinheit in Form eines Mundstücks oder einer Atemmaske, mit dem Y-Stück 22 verbunden |
| 22 | Y-Stück, welches die patientenseitige Koppeleinheit 21 mit der Inspirationsleitung 32 für die Zufuhr (Einatmung, Inspiration) des atembaren Gasgemischs Gg und der Exspirationsleitung 33 für die Abfuhr (Ausatmung, Exspiration) des ausgeatmeten Gasgemischs At verbindet |
| 23a | Rückschlagventil, das in der Inspirationsleitung 32 einen Gasfluss in Richtung des Patienten P durchlässt und in Gegenrichtung gesperrt |
| 23b | Rückschlagventil, das in der Exspirationsleitung 33 einen Gasfluss weg vom Patienten P durchlässt und in Richtung zum Patienten P hin sperrt |
| 24a | Fluidfördereinheit in Form eines Gebläses, welches einen Volumenfluss des atembaren Gasgemischs Gg in Richtung des Patienten P erzeugt |
| 24b | ansteuerbares Ventil, welches die Beatmungshübe erzeugt |
| 24c | PEEP-Ventil, welches einen end-exspiratorischen Druck in der Lunge des Patienten P aufrechterhält |
| 25a | Kohlendioxidabsorber, absorbiert aus dem Beatmungskreislauf 40 Kohlendioxid |
| 26 | Atembeutel, über den der Beatmungskreislauf 40 angetrieben werden kann |
| 27 | Fluidstrom von Frischluft oder sonstigem Frischgas zum Beatmungskreislauf 40 |
| 28 | Fluidstrom von dampfförmigem Narkosemittel zum Beatmungskreislauf 40, vom Narkosemitteldosierer 31 erzeugt |
| 29 | einstellbares Überdruckventil, welches Gas aus dem Beatmungskreislauf 40 abzulassen vermag |
| 30 | Signalverarbeitungseinheit für die Sensor-Anordnung 50, wertet Signale von den Sensoren 53, 54, 57 und 58 aus |
| 31 | Narkosemitteldosierer, erzeugt den Narkosemittelstrom 28 |
| 32 | Inspirationsleitung für die Einatmung, mit dem Y-Stück 22 verbunden, weist das Rückschlagventil 23a auf |
| 33 | Exspirationsleitung für die Ausatmung, mit dem Y-Stück 22 verbunden, weist das Rückschlagventil 23b auf |
| 34 | Abzweigpunkt des Entnahmeschlauchs 52, nahe dem Y-Stück 22 angeordnet |
| 35 | Beatmungs-Steuergerät, steuert die Fluidfördereinheit 24a, das Ventil 24b und den Narkosemitteldosierer 31 an, empfängt Signale über die jeweilige Gaskonzentration und Nachrichten von der Signalverarbeitungseinheit 30 |
| 36 | Drucksensor im Beatmungskreislauf 40, misst bevorzugt den am Patienten P anliegenden Druck $P_{aw}$ |
| 37 | Einmündepunkt, in dem der Abführschlauch 56 in den Beatmungskreislauf 40 mündet, flussaufwärts vom Kohlendioxidabsorber 25a angeordnet |
| 39 | Temperatursensor, misst die Temperatur $Temp_{amb}$ in der Umgebung des Beatmungsgeräts 1 |
| 40 | Beatmungskreislauf, durch den der Patient P künstlich beatmet wird, umfasst die patientenseitige Koppeleinheit 21, das Y-Stück 22, die Inspirationsleitung 32 und die Exspirationsleitung 33 |
| 42 | Leistungsverstärker zwischen der Spannungsquelle 43 und der Feldspule 5 |
| 43 | Spannungsquelle, gibt eine sinusförmige elektrische Spannung ab |
| 44 | optionale Anzeigeeinrichtung des Sensors 53 |

(fortgesetzt)

| | |
|---|---|
| 50 | Sensor-Anordnung, umfasst die Sensoren 53, 54, 57, den Dichtesensor 59, 60 und die Pumpe 55 |
| 51 | Wasserfalle flussaufwärts von der Sensor-Anordnung 50 |
| 52 | Entnahmeschlauch, durch den hindurch die Gasprobe Gp aus dem Beatmungskreislauf 40 entnommen wird, beginnt in einem Abzweigpunkt 34 zwischen der patientenseitigen Koppeleinheit 21 und dem Y-Stück 22 und führt zu der Wasserfalle 51 |
| 53 | Sensor für die Konzentration von Sauerstoff |
| 54 | Sensor für die Konzentration von $CO_2$, $N_2O$ und Narkosemittel in der Gasprobe Gp |
| 55 | Pumpe, welche eine Gasprobe Gp in den Entnahmeschlauch 52 einsaugt |
| 56 | Abführschlauch, verbindet die Sensor-Anordnung 50 mit dem Beatmungskreislauf 40 |
| 57 | Drucksensor der Sensor-Anordnung 50, misst den Druck der Gasprobe Gp |
| 58 | Sensor für den Umgebungsdruck $P_{amb}$ |
| 59 | mechanischer Biegeschwinger, der die Dichte der Gasprobe Gp misst, umfasst das Gehäuse 83, das Rohr 80 mit den Schenkeln 80.1, 80.2, die Befestigungsstelle 81, den elektromagnetischen Umwandler 82.1, 82.2, das Stellglied 84, den Verstärker 85 und die Auswerteeinheit 86 |
| 60 | elektronischer Biegeschwinger in Form eines Schwingquarzes, umfasst die Schenkel 61.1, 61.2 |
| 62 | Elektromagnet, erzeugt ein zeitlich veränderliches Magnetfeld im Luftspalt 3, umfasst die Spule 63 |
| 63 | elektrische Spule des Elektromagneten 62 |
| 64 | Messeinheit, als Chip implementiert, umfasst das Wärmeleitungs-Messelement 65, die Heizeinheit 66 und die Membrane 67 |
| 65 | elektrisch steuerbares Wärmeleitungs-Messelement der Messeinheit 64 |
| 66 | elektrisch steuerbare Heizeinheit der Messeinheit 64 |
| 67 | Membrane der Messeinheit 64 |
| 68 | elektrisch leitendes Verbindungselement zwischen den beiden Heizelementen der Heizeinheit 66 |
| 69 | Rahmen der Messeinheit 64 |
| 70 | Verstärker |
| 71 | Spannungsteiler |
| 72 | Gleichspannungsquelle |
| 73 | Tiefpass-Filter, liefert an seinem Ausgang das Signal 76 |
| 74 | Hochpass-Filter, liefert an seinem Ausgang das Signal 75 |
| 75 | Signal am Ausgang des Hochpass-Filters 74, korreliert mit der Konzentration $\xi(O_2)$ von Sauerstoff |
| 76 | Signal am Ausgang des Tiefpass-Filters 73, korreliert mit der Wärmeleitfähigkeit $\lambda$ der Gasprobe Gp |
| 77 | Messposition der Messeinheit 64 |
| 80 | U-förmiges Rohr, hat die beiden Schenkel 80.1, 80.2, nimmt die Gasprobe Gp auf, wird zu Schwingungen angeregt |
| 80.1, 80.2 | Schenkel des U-förmigen Rohrs 80 |
| 81 | Befestigungsstelle, an der die beiden Schenkel 80.1, 80.2 eingespannt sind |
| 82.1, 82.2 | elektromagnetische Umwandler, wandeln in einem Modus elektrische Wechselspannung in mechanische Schwingungen um und in einem anderen Modus mechanische Schwingungen in elektrische Wechselspannung, umfassen die Spulen 83.1, 83.2 und die Magneten 84.1, 84.2 |
| 83 | Gehäuse des Dichtesensors 59, nimmt das Rohr 80 auf |
| 83.1, 83.2 | Spule des Umwandlers 82.1, 82.2 |
| 84.1, 84.2 | Magnet des Umwandlers 82.1, 82.2 |
| 85 | Verstärker des Dichtesensors 59 |

(fortgesetzt)

| 86 | Auswerteeinheit des Dichtesensors 59 |
|---|---|
| 100 | Messsystem, umfasst die Sensor-Anordnung 50, die Wasserfalle 51, die Signalverarbeitungseinheit 30 und die Schläuche 52 und 56 |
| 200 | Beatmungs-Anordnung, umfasst das Anästhesiegerät 1, das Messsystem 100, die patientenseitige Koppeleinheit 21 und die Leitungen 32 und 33 |
| At | vom Patienten P ausgeatmetes Gasgemisch, enthält Kohlendioxid und ein Narkosemittel |
| G(i,k) | empirisch bestimmte Gewichtsfaktoren |
| Gg | atembares Gasgemisch, welches das Beatmungsgerät 1 zur patientenseitigen Koppeleinheit 21 fördert, enthält Sauerstoff, ein Narkosemittel und Argon |
| Gp | Gasprobe, wird aus dem Beatmungskreislauf 40 abgezweigt, durch den Entnahmeschlauch 52 hindurch zur Sensor-Anordnung 50 geleitet und durch den Abführschlauch 56 wieder in den Beatmungskreislauf 40 eingespeist |
| $\lambda$ | Wärmeleitfähigkeit der Gasprobe Gp |
| $\lambda(x_i)$ | Wärmeleitfähigkeit des Gasbestandteils $x_i$ (i=1, ... ,n) der Gasprobe Gp |
| $\lambda_{2f}$ | magnetisch modulierte Wärmeleitfähigkeit, ist ein Maß für die Sauerstoff-Konzentration in der Gasprobe Gp |
| $\lambda_{2f}(x_i)$ | magnetisch modulierte Wärmeleitfähigkeit des Gasbestandteils $x_i$ (i=1, ..., n) der Gasprobe Gp |
| n | Anzahl der Gasbestandteile in der Gasprobe Gp |
| P | Patient, der künstlich beatmet wird und mit der patientenseitigen Koppeleinheit 21 verbunden ist |
| $P_{amb}$ | Druck in der Umgebung der Beatmungs-Anordnung 200, vom Drucksensor 58 gemessen |
| $P_{aw}$ | Atemwegsdruck in der patientenseitigen Koppeleinheit 21, wird vom Drucksensor 36 in der Inspirationsleitung 32 gemessen |
| $P_{cell}$ | Druck der Gasprobe Gp am Eingang der Sensor-Anordnung 50, vom Drucksensor 57 gemessen |
| $\rho$ | Dichte der Gasprobe Gp |
| $\rho(x_i)$ | Dichte des Gasbestandteils $x_i$ (i=1, ..., n) der Gasprobe Gp |
| $Temp_{amb}$ | Umgebungstemperatur, vom Temperatursensor 39 gemessen |
| $\xi(x_i)$ | Konzentration in Vol.-% des Gasbestandteils $x_i$ (i=1, ..., n) der Gasprobe Gp |
| $x_1, ..., x_n$ | Gasbestandteile der Gasprobe Gp |

**Patentansprüche**

1. Sensor-Anordnung (50) zum Messen der jeweiligen Konzentration von drei Gasbestandteilen einer Gasprobe (Gp),

   wobei die jeweilige Dichte und die jeweilige Wärmeleitfähigkeit jeder der drei Gasbestandteile vorgegeben sind,
   wobei ein Gasbestandteil ($O_2$) der drei Gasbestandteile ein paramagnetisches Gas ist,
   wobei die Sensor-Anordnung (50)

   - eine Messkammer (2) zum Aufnehmen einer Gasprobe (Gp),
   - einen Wärmeleitfähigkeits-Sensor (53) und
   - einen Dichtesensor (59, 60)

   umfasst,
   wobei der Wärmeleitfähigkeits-Sensor (53) dazu ausgestaltet ist, die Wärmeleitfähigkeit ($\lambda$) der Gasprobe (Gp) zu messen,
   wobei die Sensor-Anordnung (50) dazu ausgestaltet ist,

   - die Gasprobe (Gp) in die Messkammer (2) zu leiten,

- an die Messkammer (2) ein Magnetfeld mit einer oszillierenden Magnetfeldstärke anzulegen und
- eine magnetisch modulierte Wärmeleitfähigkeit ($\lambda_{2f}$) der Gasprobe (Gp) in der Messkammer (2) zu messen,

wobei die magnetisch modulierte Wärmeleitfähigkeit ($\lambda_{2f}$) derjenige Anteil der Wärmeleitfähigkeit der Gasprobe (Gp) ist, der abhängig von der Magnetfeldstärke oszilliert,
wobei der Dichtesensor (59, 60) dazu ausgestaltet ist, die Dichte ($\rho$) der Gasprobe (Gp) zu messen, und
wobei die Sensor-Anordnung (50) dazu ausgestaltet ist,
die drei Gasbestandteil-Konzentrationen unter Verwendung

- der vorgegebenen Dichten der Gasbestandteile,
- der vorgegebenen Wärmeleitfähigkeiten der Gasbestandteile,
- der gemessenen Wärmeleitfähigkeit ($\lambda$) der Gasprobe (Gp),
- der gemessenen magnetisch modulierten Wärmeleitfähigkeit ($\lambda_{2f}$) der Gasprobe (Gp) und
- der gemessenen Dichte ($\rho$) der Gasprobe (Gp)

zu ermitteln.

2. Sensor-Anordnung (50) nach Anspruch 1,
   **dadurch gekennzeichnet, dass**

   die Sensor-Anordnung (50) eine Heizeinheit (8, 66) umfasst,
   wobei die Heizeinheit (8, 66) dazu ausgestaltet ist, einer Gasprobe (Gp) in der Messkammer (2) Wärmeenergie zuzuführen,
   wobei die Sensor-Anordnung (50) dazu ausgestaltet ist,

   - eine elektrische Detektionsgröße (U) zu messen, welche mit der Wärmeleitfähigkeit ($\lambda$) der Gasprobe (Gp) korreliert, und
   - mittels einer Filterung der elektrischen Detektionsgröße (U) ein oszillierendes Signal (75), das abhängig von der Magnetfeldstärke oszilliert, und ein weiteres Signal (76), dessen zeitlicher Verlauf nicht von der oszillierenden Magnetfeldstärke abhängt, herzuleiten, und

   wobei die Sensor-Anordnung (50) weiterhin dazu ausgestaltet ist,

   - die magnetisch modulierte Wärmeleitfähigkeit ($\lambda_{2f}$) der Gasprobe (Gp) abhängig vom oszillierenden Signal (75) zu messen, insbesondere das oszillierende Signal (75) als die magnetisch modulierte Wärmeleit-fähigkeit ($\lambda_{2f}$) der Gasprobe (Gp) zu verwenden, und
   - die Wärmeleitfähigkeit ($\lambda$) der Gasprobe (Gp) abhängig vom weiteren Signal (76) zu messen, insbesondere das weitere Signal (76) als die Wärmeleitfähigkeit ($\lambda$) zu verwenden.

3. Sensor-Anordnung (50) nach einem der vorhergehenden Ansprüche,
   **dadurch gekennzeichnet, dass**

   der Dichtesensor einen Biegeschwinger (59, 60) mit einem Schwingkörper (80, 61.1, 61.2) umfasst,
   wobei die Sensor-Anordnung (50) dazu ausgestaltet ist, die Gasprobe (Gp) durch den oder entlang des Schwingkörpers (80, 61.1, 61.2) zu leiten,
   wobei der Biegeschwinger (59, 60) dazu ausgestaltet ist,

   - den Schwingkörper (80, 61.1, 61.2) in Schwingungen zu versetzen und
   - die Eigenfrequenz des schwingenden Schwingkörpers (80, 61.1, 61.2) zu messen, und

   wobei die Sensor-Anordnung (50) dazu ausgestaltet ist, die Dichte ($\rho$) der Gasprobe (Gp) abhängig von der gemessenen Eigenfrequenz des Schwingkörpers (80, 61.1, 61.2) zu ermitteln.

4. Beatmungs-Anordnung (200) zur künstlichen Beatmung eines Patienten (P)

   wobei die Beatmungs-Anordnung (200)

   - ein medizinisches Gerät (1), insbesondere ein Beatmungsgerät,

- eine patientenseitige Koppeleinheit (21),
- ein Beatmungs-Steuergerät (35) und
- eine Sensor-Anordnung (50) nach einem der vorhergehenden Ansprüche

umfasst,

wobei die patientenseitige Koppeleinheit (21) mit dem Patienten (P) verbunden oder wenigstens zeitweise verbindbar ist,

wobei die Beatmungs-Anordnung (200) dazu ausgestaltet ist, ein atembares Gasgemisch (Gg) vom medizinischen Gerät (1) zur patientenseitigen Koppeleinheit (21) zu fördern,

wobei ein Sollbereich für die Konzentration [$\xi(O_2)$] eines Gasbestandteils ($O_2$) im Gasgemisch (Gg) vorgegeben ist,

wobei bevorzugt dieser Gasbestandteil das paramagnetische Gas ist,

wobei die Beatmungs-Anordnung (200) dazu ausgestaltet ist,

- eine Gasprobe (Gp) aus dem Gasgemisch (Gg), das zur patientenseitigen Koppeleinheit (21) gefördert wird, abzuzweigen und
- die abgezweigte Gasprobe (Gp) zur Sensor-Anordnung (50) zu leiten, und

wobei die Sensor-Anordnung (50) dazu ausgestaltet ist, die tatsächliche Konzentration [$\xi(O_2)$] des Gasbestandteils ($O_2$), für den der Sollbereich vorgegeben ist, in der abgezweigten Gasprobe (Gp) zu messen.

5. Beatmungs-Anordnung (200) nach Anspruch 4,
**dadurch gekennzeichnet, dass**

das Beatmungs-Steuergerät (35) dazu ausgestaltet ist,

- die Konzentration [$\xi(O_2)$] des Gasbestandteils ($O_2$), für den der Sollbereich vorgegeben ist, im Gasgemisch (Gg), das zur patientenseitigen Koppeleinheit (21) gefördert wird, zu regeln und
- für die Regelung die gemessene Gasbestandteil-Konzentration [$\xi(O_2)$] zu verwenden, und

wobei ein Regelungsziel bei der Regelung ist, dass die tatsächliche Gasbestandteil-Konzentration [$\xi(O_2)$] im Gasgemisch (Gg) in dem vorgegebenen Sollbereich bleibt.

6. Mess-Verfahren zum Messen der jeweiligen Konzentration von drei Gasbestandteilen einer Gasprobe (Gp),

wobei die jeweilige Dichte und die jeweilige Wärmeleitfähigkeit jedes der drei Gasbestandteile vorgegeben werden,

wobei ein Gasbestandteil ($O_2$) der drei Gasbestandteile ein paramagnetisches Gas ist und

wobei das Mess-Verfahren die Schritte umfasst, dass

- die Wärmeleitfähigkeit der Gasprobe (Gp) gemessen wird und
- die Dichte der Gasprobe (Gp) gemessen wird,

wobei das Mess-Verfahren die weiteren Schritte umfasst, dass

- die Gasprobe (Gp) in eine Messkammer (2) geleitet wird,
- an die Messkammer (2) ein Magnetfeld mit einer oszillierenden Magnetfeldstärke angelegt wird und
- eine magnetisch modulierte Wärmeleitfähigkeit ($\lambda_{2f}$) der Gasprobe (Gp) in der Messkammer (2) gemessen wird,

wobei die magnetisch modulierte Wärmeleitfähigkeit ($\lambda_{2f}$) derjenige Anteil der Wärmeleitfähigkeit der Gasprobe (Gp) ist, der abhängig von der Magnetfeldstärke oszilliert, und

wobei der weitere Schritt durchgeführt wird, dass
die drei Gasbestandteil-Konzentrationen unter Verwendung

- der vorgegebenen Dichten der Gasbestandteile,
- der vorgegebenen Wärmeleitfähigkeiten der Gasbestandteile,
- der gemessenen Wärmeleitfähigkeit ($\lambda$) der Gasprobe (Gp),

- der gemessenen magnetisch modulierten Wärmeleitfähigkeit ($\lambda_{2f}$) der Gasprobe (Gp) und
- der gemessenen Dichte ($\rho$) der Gasprobe (Gp)

ermittelt werden.

**7.** Mess-Verfahren nach Anspruch 6,
**dadurch gekennzeichnet, dass**

als die Wärmeleitfähigkeit ($\lambda$) die Wärmeleitfähigkeit der Gasprobe (Gp) in der Messkammer (2) gemessen wird und
das Mess-Verfahren die weiteren Schritte umfasst, dass

- eine Heizeinheit (8, 66) erhitzt wird,
- die erhitzte Heizeinheit (8, 66) der Gasprobe (Gp) in der Messkammer (2) Wärmeenergie zuführt,
- eine elektrische Detektionsgröße (U) der erhitzten Heizeinheit (8, 66) gemessen wird,
wobei die Detektionsgröße (U) mit der Wärmeleitfähigkeit ($\lambda$) der Gasprobe (Gp) korreliert, und
- mittels einer Filterung der elektrischen Detektionsgröße (U) ein oszillierendes Signal (75), das abhängig von der Magnetfeldstärke oszilliert, und ein weiteres Signal (76), dessen zeitlicher Verlauf nicht von der oszillierenden Magnetfeldstärke abhängt, hergeleitet werden,

wobei

- die magnetisch modulierte Wärmeleitfähigkeit ($\lambda_{2f}$) der Gasprobe (Gp) abhängig vom oszillierenden Signal (75) gemessen wird,
insbesondere das oszillierende Signal (75) als die magnetisch modulierte Wärmeleitfähigkeit ($\lambda_{2f}$) der Gasprobe (Gp) verwendet wird, und
- die Wärmeleitfähigkeit ($\lambda$) der Gasprobe (Gp) abhängig vom weiteren Signal (76) gemessen wird,
insbesondere das weitere Signal (76) als die Wärmeleitfähigkeit ($\lambda$) verwendet wird.

**8.** Mess-Verfahren nach Anspruch 6 oder Anspruch 7,
**dadurch gekennzeichnet, dass**

das Mess-Verfahren die weiteren Schritte umfasst, dass

- die Gasprobe (Gp) durch einen oder entlang eines Schwingkörpers (80, 61.1, 61.2) eines Biegeschwingers (59, 60) geleitet wird,
- der Schwingkörper (80, 61.1, 61.2) in Schwingungen versetzt wird und
- die Eigenfrequenz des schwingenden Schwingkörpers (80, 61.1, 61.2) gemessen wird,

wobei die Dichte der Gasprobe (Gp) abhängig von der gemessenen Eigenfrequenz des Schwingkörpers (80, 61.1, 61.2) ermittelt wird.

**9.** Mess-Verfahren nach einem der Ansprüche 6 bis 8,
**dadurch gekennzeichnet, dass**

die Wärmeleitfähigkeit, die magnetisch modulierte Wärmeleitfähigkeit und / oder die Dichte der Gasprobe (Gp) zusätzlich von der Konzentration eines vierten Gasbestandteils abhängt,
wobei die Dichte des vierten Gasbestandteils vorgegeben wird und
wobei das Mess-Verfahren die weiteren Schritte umfasst, dass

- die Konzentration des vierten Gasbestandteils gemessen oder auf andere Weise ermittelt wird und
- beim Schritt, die jeweilige Konzentration der drei Gasbestandteile zu ermitteln, zusätzlich die gemessene Konzentration des vierten Gasbestandteils verwendet wird.

**10.** Mess-Verfahren nach Anspruch 9,
**dadurch gekennzeichnet, dass**

der Schritt, die Konzentration des vierten Gasbestandteils zu messen,

die Schritte umfasst, dass

- eine Strahlungsquelle elektromagnetische Strahlung oder Schall emittiert,
- die emittierte elektromagnetische Strahlung oder der emittierte Schall die Gasprobe (Gp) durchdringt und auf einen Detektor auftrifft,
- der Detektor die Intensität der auftreffenden elektromagnetischen Strahlung oder des auftreffenden Schalls misst und
- der Detektor abhängig von der gemessenen Intensität ein Signal generiert,

wobei die Konzentration des vierten Gasbestandteils abhängig von dem vom Detektor generierten Signal gemessen wird,
insbesondere das vom Detektor generierte Signal als die Konzentration des vierten Gasbestandteils verwendet wird.

**11.** Mess-Verfahren nach einem der Ansprüche 6 bis 10,
**dadurch gekennzeichnet, dass**

drei funktionale Zusammenhänge, drei Abstandsmaße und eine Zielfunktion vorgegeben werden,
wobei der erste funktionale Zusammenhang die Wärmeleitfähigkeit ($\lambda$) der Gasprobe (Gp) als Funktion der drei Gasbestandteil-Konzentrationen beschreibt,
wobei der zweite funktionale Zusammenhang die magnetisch modulierte Wärmeleitfähigkeit ($\lambda_{2f}$) der Gasprobe (Gp) als Funktion der drei Gasbestandteil-Konzentrationen beschreibt,
wobei der dritte funktionale Zusammenhang die Dichte ($\rho$) der Gasprobe (Gp) als Funktion der drei Gasbestandteil-Konzentrationen beschreibt,
wobei das erste Abstandsmaß ein Maß für einen Abstand zwischen der gemessenen Wärmeleitfähigkeit ($\lambda$) der Gasprobe (Gp) und der Wärmeleitfähigkeit, die mit Hilfe des ersten funktionalen Zusammenhangs hergeleitet wird, beschreibt,
wobei das zweite Abstandsmaß ein Maß für einen Abstand zwischen der gemessenen magnetisch modulierten Wärmeleitfähigkeit ($\lambda_{2f}$) der Gasprobe (Gp) und der magnetisch modulierten Wärmeleitfähigkeit, die mit Hilfe des zweiten funktionalen Zusammenhangs hergeleitet wird, beschreibt,
wobei das dritte Abstandsmaß ein Maß für einen Abstand zwischen der gemessenen Dichte ($\rho$) der Gasprobe (Gp) und der Dichte, die mit Hilfe des dritten funktionalen Zusammenhangs hergeleitet wird, beschreibt,
wobei die Zielfunktion eine Aggregation, insbesondere ein gewichtetes Mittel, der drei Abstandsmaße ist und
wobei das Mess-Verfahren die weiteren Schritte umfasst, dass

- die drei gemessenen Werte für die Wärmeleitfähigkeit ($\lambda$), die magnetisch modulierte Wärmeleitfähigkeit ($\lambda_{2f}$) und die Gasproben-Dichte ($\rho$) in die Zielfunktion eingesetzt werden und
- ein Werte-Tripel für die drei Gasbestandteil-Konzentrationen dergestalt berechnet wird, dass die Zielfunktion minimiert wird.

**12.** Mess-Verfahren nach einem der Ansprüche 6 bis 11,
**dadurch gekennzeichnet, dass**
die drei Gasbestandteile, deren Konzentrationen in der abgezweigten Gasprobe (Gp) gemessen werden, Sauerstoff ($O_2$), ein Narkosemittel und Argon sind.

**13.** Beatmungs-Verfahren zur künstlichen Beatmung eines Patienten (P),

wobei der Patient (P) mit einer patientenseitigen Koppeleinheit (21) verbunden wird oder ist und
wobei das Beatmungs-Verfahren die automatisch durchgeführten Schritte umfasst, dass

- ein atembares Gasgemisch (Gg) von einem medizinischen Gerät (1), insbesondere von einem Beatmungsgerät, zur patientenseitigen Koppeleinheit (21) gefördert wird,
- ein Sollbereich für die Konzentration [$\xi(O_2)$] eines Gasbestandteils ($O_2$) im Gasgemisch (Gg) vorgegeben ist,
- eine Gasprobe (Gp) aus dem Gasgemisch (Gg), das zur patientenseitigen Koppeleinheit (21) gefördert wird, abgezweigt wird,
- die tatsächliche Konzentration [$\xi(O_2)$] des Gasbestandteils ($O_2$), für das der Sollbereich vorgegeben ist, gemessen wird,

wobei für die Messung der tatsächlichen Gasbestandteil-Konzentration [ξ(O₂)] ein Mess-Verfahren nach einem der Ansprüche 6 bis 12 angewendet wird und

wobei der Gasbestandteil, für den der Sollbereich vorgegeben ist, eines der drei Gasbestandteile ist, deren Konzentration gemessen wird, bevorzugt das paramagnetische Gas,

- wobei bevorzugt die tatsächliche Konzentration [ξ(O₂)] des Gasbestandteils (O₂) im Gasgemisch (Gg) mit dem Regelungsziel geregelt wird, dass die tatsächliche Sauerstoffkonzentration im vorgegebenen Sollbereich liegt,

wobei für die Regelung die gemessene tatsächliche Gasbestandteil-Konzentration [ξ(O₂)] in der abgezweigten Gasprobe (Gp) verwendet wird.

14. Beatmungs-Verfahren nach Anspruch 13,
**dadurch gekennzeichnet, dass**

das Gasgemisch (Gg) unter Verwendung von Umgebungsluft erzeugt wird,

wobei bei der Erzeugung des Gasgemischs (Gg) die Sauerstoffkonzentration vergrößert wird und

wobei die drei Gasbestandteile, deren Konzentrationen in der abgezweigten Gasprobe (Gp) gemessen werden, Sauerstoff (O₂), ein Narkosemittel und Argon sind.

15. Beatmungs-Verfahren nach Anspruch 13 oder Anspruch 14,
**dadurch gekennzeichnet, dass**

das Gasgemisch (Gg) durch eine Fluidführungseinheit (32) hindurch vom medizinischen Gerät (1) zur patientenseitigen Koppeleinheit (21) gefördert wird und

die Detektion des Ereignisses, dass mindestens ein Gasbestandteil im geförderten Gasgemisch (Gg) außerhalb des für diesen Gasbestandteil vorgegebenen Sollbereichs liegt,

den Schritt auslöst, dass die Fluidführungseinheit (32) gespült wird.

**Claims**

1. Sensor arrangement (50) for measuring the particular concentration of three gas components of a gas sample (Gp),

wherein the particular density and the particular thermal conductivity of each of the three gas components are specified,

wherein one gas component (O₂) of the three gas components is a paramagnetic gas,

wherein the sensor arrangement (50) comprises

- a measuring chamber (2) for receiving a gas sample (Gp),
- a thermal conductivity sensor (53) and
- a density sensor (59, 60),

wherein the thermal conductivity sensor (53) is configured to measure the thermal conductivity (A) of the gas sample (Gp),

wherein the sensor arrangement (50) is configured to

- guide the gas sample (Gp) into the measuring chamber (2),
- apply a magnetic field having an oscillating magnetic field strength to the measuring chamber (2) and
- measure a magnetically modulated thermal conductivity ($\lambda_{2f}$) of the gas sample (Gp) in the measuring chamber (2),

wherein the magnetically modulated thermal conductivity ($\lambda_{2f}$) is the portion of the thermal conductivity of the gas sample (Gp) that oscillates depending on the magnetic field strength,

wherein the density sensor (59, 60) is configured to measure the density ($\rho$) of the gas sample (Gp), and

wherein the sensor arrangement (50) is configured to

determine the three gas component concentrations using

- the specified densities of the gas components,

- the specified thermal conductivities of the gas components,
- the measured thermal conductivity ($\lambda$) of the gas sample (Gp),
- the measured magnetically modulated thermal conductivity ($\lambda_{2f}$) of the gas sample (Gp) and
- the measured density ($\rho$) of the gas sample (Gp).

2. Sensor arrangement (50) according to claim 1,
**characterized in that**

the sensor arrangement (50) comprises a heating unit (8, 66),
the heating unit (8, 66) being configured to supply thermal energy to a gas sample (Gp) in the measuring chamber (2),
the sensor arrangement (50) being configured to

- measure an electrical detection quantity (U) which correlates with the thermal conductivity ($\lambda$) of the gas sample (Gp), and
- derive an oscillating signal (75), which oscillates depending on the magnetic field strength, and a further signal (76), of which the time course does not depend on the oscillating magnetic field strength, by filtering the electrical detection quantity (U), and

the sensor arrangement (50) being further configured to

- measure the magnetically modulated thermal conductivity ($\lambda_{2f}$) of the gas sample (Gp) depending on the oscillating signal (75), in particular to use the oscillating signal (75) as the magnetically modulated thermal conductivity ($\lambda_{2f}$) of the gas sample (Gp), and
- measure the thermal conductivity ($\lambda$) of the gas sample (Gp) depending on the further signal (76), in particular to use the further signal (76) as the thermal conductivity (A).

3. Sensor arrangement (50) according to either of the preceding claims,
**characterized in that**

the density sensor comprises a flexural resonator (59, 60) having a vibrating body (80, 61.1, 61.2),
the sensor arrangement (50) being configured to guide the gas sample (Gp) through or along the vibrating body (80, 61.1, 61.2),
the flexural resonator (59, 60) being configured to

- set the vibrating body (80, 61.1, 61.2) into vibrations and
- measure the natural frequency of the vibrating body (80, 61.1, 61.2), and

the sensor arrangement (50) being configured to determine the density ($\rho$) of the gas sample (Gp) depending on the measured natural frequency of the vibrating body (80, 61.1, 61.2).

4. Ventilation arrangement (200) for artificial ventilation of a patient (P),

wherein the ventilation arrangement (200) comprises

- a medical device (1), in particular a ventilator,
- a patient-side coupling unit (21),
- a ventilation control device (35) and
- a sensor arrangement (50) according to any of the preceding claims,

wherein the patient-side coupling unit (21) is connected or at least temporarily connectable to the patient (P),
wherein the ventilation arrangement (200) is configured to convey a breathable gas mixture (Gg) from the medical device (1) to the patient-side coupling unit (21),
wherein a target range for the concentration [$\xi(O_2)$] of a gas component ($O_2$) in the gas mixture (Gg) is specified,
wherein preferably this gas component is the paramagnetic gas,
wherein the ventilation arrangement (200) is configured to

- divert a gas sample (Gp) from the gas mixture (Gg) that is conveyed to the patient-side coupling unit (21) and

- guide the diverted gas sample (Gp) to the sensor arrangement (50), and

wherein the sensor arrangement (50) is configured to measure the actual concentration $[\xi(O_2)]$ of the gas component $(O_2)$ for which the target range is specified in the diverted gas sample (Gp).

5. Ventilation arrangement (200) according to claim 4,
   **characterized in that**

   the ventilation control device (35) is configured to

   - regulate the concentration $[\xi(O_2)]$ of the gas component $(O_2)$ for which the target range is specified in the gas mixture (Gg) that is conveyed to the patient-side coupling unit (21) and
   - use the measured gas component concentration $[\xi(O_2)]$ for the regulation, and

   one regulation objective in the course of the regulation being that the actual gas component concentration $[\xi(O_2)]$ in the gas mixture (Gg) remains in the specified target range.

6. Measuring method for measuring the particular concentration of three gas components of a gas sample (Gp),

   wherein the particular density and the particular thermal conductivity of each of the three gas components are specified,
   wherein one gas component $(O_2)$ of the three gas components is a paramagnetic gas and
   wherein the measuring method comprises the steps in which

   - the thermal conductivity of the gas sample (Gp) is measured and
   - the density of the gas sample (Gp) is measured,

   wherein the measuring method comprises the further steps in which

   - the gas sample (Gp) is guided into a measuring chamber (2),
   - a magnetic field having an oscillating magnetic field strength is applied to the measuring chamber (2) and
   - a magnetically modulated thermal conductivity $(\lambda_{2f})$ of the gas sample (Gp) is measured in the measuring chamber (2),

   wherein the magnetically modulated thermal conductivity $(\lambda_{2f})$ is the portion of the thermal conductivity of the gas sample (Gp) that oscillates depending on the magnetic field strength, and
   wherein the further step is performed in which
   the three gas component concentrations are determined using

   - the specified densities of the gas components,
   - the specified thermal conductivities of the gas components,
   - the measured thermal conductivity (A) of the gas sample (Gp),
   - the measured magnetically modulated thermal conductivity $(\lambda_{2f})$ of the gas sample (Gp) and
   - the measured density (p) of the gas sample (Gp).

7. Measuring method according to claim 6,
   **characterized in that**

   the thermal conductivity of the gas sample (Gp) in the
   measuring chamber (2) is measured as the thermal conductivity (A) and
   the measuring method comprises the further steps in which

   - a heating unit (8, 66) is heated,
   - the heated heating unit (8, 66) supplies thermal energy to the gas sample (Gp) in the measuring chamber (2),
   - an electrical detection quantity (U) of the heated heating unit (8, 66) is measured,

   the detection quantity (U) correlating with the thermal conductivity $(\lambda)$ of the gas sample (Gp), and

- an oscillating signal (75), which oscillates depending on the magnetic field strength, and a further signal (76), of which the time course does not depend on the oscillating magnetic field strength, are derived by filtering the electrical detection quantity (U),
- the magnetically modulated thermal conductivity ($\lambda_{2f}$) of the gas sample (Gp) being measured depending on the oscillating signal (75),

in particular the oscillating signal (75) being used as the magnetically modulated thermal conductivity ($\lambda_{2f}$) of the gas sample (Gp), and

- the thermal conductivity ($\lambda$) of the gas sample (Gp) being measured depending on the further signal (76),

in particular the further signal (76) being used as the thermal conductivity ($\lambda$).

8. Measuring method according to claim 6 or claim 7,
   **characterized in that**

   the measuring method comprises the further steps in which

   - the gas sample (Gp) is guided through or along a vibrating body (80, 61.1, 61.2) of a flexural resonator (59, 60),
   - the vibrating body (80, 61.1,61.2) is set into vibrations and
   - the natural frequency of the vibrating body (80, 61.1,61.2) is measured,

   the density of the gas sample (Gp) being determined depending on the measured natural frequency of the vibrating body (80, 61.1,61.2).

9. Measuring method according to any of claims 6 to 8,
   **characterized in that**

   the thermal conductivity, the magnetically modulated thermal conductivity and/or the density of the gas sample (Gp) additionally depends on the concentration of a fourth gas component,
   the density of the fourth gas component being specified and
   the measuring method comprising the further steps in which

   - the concentration of the fourth gas component is measured or is determined in some other way and
   - in the step of determining the particular concentration of the three gas components, the measured concentration of the fourth gas component is additionally used.

10. Measuring method according to claim 9,
    **characterized in that**

    the step of measuring the concentration of the fourth gas component
    comprises the steps in which

    - a radiation source emits electromagnetic radiation or sound,
    - the emitted electromagnetic radiation or the emitted sound penetrates the gas sample (Gp) and strikes a detector,
    - the detector measures the intensity of the incident electromagnetic radiation or the incident sound and
    - the detector generates a signal depending on the measured intensity,

    the concentration of the fourth gas component being measured depending on the signal generated by the detector,
    in particular the signal generated by the detector being used as the concentration of the fourth gas component.

11. Measuring method according to any of claims 6 to 10,
    **characterized in that**

    three functional relationships, three distance indicators, and one objective function are specified,

the first functional relationship describing the thermal conductivity ($\lambda$) of the gas sample (Gp) as a function of the three gas component concentrations,

the second functional relationship describing the magnetically modulated thermal conductivity ($\lambda_{2f}$) of the gas sample (Gp) as a function of the three gas component concentrations,

the third functional relationship describing the density (p) of the gas sample (Gp) as a function of the three gas component concentrations,

the first distance indicator describing an indicator for the distance between the measured thermal conductivity ($\lambda$) of the gas sample (Gp) and the thermal conductivity derived using the first functional relationship,

the second distance indicator describing an indicator for the distance between the measured magnetically modulated thermal conductivity ($\lambda_{2f}$) of the gas sample (Gp) and the magnetically modulated thermal conductivity derived using the second functional relationship,

the third distance indicator describing an indicator for the distance between the measured density (p) of the gas sample (Gp) and the density derived using the third functional relationship,

the objective function being an aggregation, in particular a weighted average, of the three distance indicators and the measuring method comprising the further steps in which

- the three measured values for the thermal conductivity ($\lambda$), the magnetically modulated thermal conductivity ($\lambda_{2f}$) and the gas sample density (p) are inserted into the objective function and
- a triple of values for the three gas component concentrations is calculated in such a way that the objective function is minimized.

**12.** Measuring method according to any of claims 6 to 11,
**characterized in that**
the three gas components of which the concentrations are measured in the diverted gas sample (Gp) are oxygen ($O_2$), an anesthetic and argon.

**13.** Ventilation method for artificial ventilation of a patient (P),

wherein the patient (P) is connected to a patient-side coupling unit (21) and
wherein the ventilation method comprises the automatically performed steps in which

- a breathable gas mixture (Gg) is conveyed from a medical device (1), in particular from a ventilator, to the patient-side coupling unit (21),

a target range for the concentration [$\xi(O_2)$] of a gas component ($O_2$) in the gas mixture (Gg) is specified,

- a gas sample (Gp) is diverted from the gas mixture (Gg) that is conveyed to the patient-side coupling unit (21),
- the actual concentration [$\xi(O_2)$] of the gas component ($O_2$) for which the target range is specified is measured,

wherein, for the measurement of the actual gas component concentration [$\xi(O_2)$], a measuring method according to any of claims 6 to 12 is applied and
wherein the gas component for which the target range is specified is one of the three gas components of which the concentration is measured, preferably the paramagnetic gas,

- wherein the actual concentration [$\xi(O_2)$] of the gas component ($O_2$) in the gas mixture (Gg) is preferably regulated using the regulation objective that the actual oxygen concentration is within the specified target range,

wherein, for the regulation, the measured gas component concentration [$\xi(O_2)$] in the diverted gas sample (Gp) is used.

**14.** Ventilation method according to claim 13,
**characterized in that**

the gas mixture (Gg) is produced using ambient air,
the oxygen concentration being increased during the production of the gas mixture (Gg) and

the three gas components of which the concentrations are measured in the diverted gas sample (Gp) being oxygen (O$_2$), an anesthetic and argon.

**15.** Ventilation method according to claim 13 or claim 14,
**characterized in that**

the gas mixture (Gg) is conveyed through a fluid guidance unit (32) from the medical device (1) to the patient-side coupling unit (21) and
the detection of the event that at least one gas component in the conveyed gas mixture (Gg) lies outside the target range specified for that gas component
triggers the step of flushing the fluid guidance unit (32).

**Revendications**

**1.** Agencement de capteurs (50) pour la mesure de la concentration respective de trois composants gazeux d'un échantillon de gaz (Gp),

dans lequel la densité respective et la conductivité thermique respective de chacun des trois composants gazeux sont prédéfinies,
dans lequel un composant gazeux (O$_2$) des trois composants gazeux est un gaz paramagnétique,
dans lequel l'agencement de capteurs (50) comprend

- une chambre de mesure (2) pour la réception d'un échantillon de gaz (Gp),
- un capteur de conductivité thermique (53) et
- un capteur de densité (59, 60),

dans lequel le capteur de conductivité thermique (53) est configuré pour mesurer la conductivité thermique ($\lambda$) de l'échantillon de gaz (Gp),
dans lequel l'agencement de capteurs (50) est configuré pour

- conduire l'échantillon de gaz (Gp) dans la chambre de mesure (2),
- appliquer à la chambre de mesure (2) un champ magnétique comportant une intensité de champ magnétique oscillante et
- mesurer une conductivité thermique à modulation magnétique ($\lambda_{2f}$) de l'échantillon de gaz (Gp) dans la chambre de mesure (2),

dans lequel la conductivité thermique à modulation magnétique ($\lambda_{2f}$) est la fraction de la conductivité thermique de l'échantillon de gaz (Gp) qui oscille en fonction de l'intensité de champ magnétique,
dans lequel le capteur de densité (59, 60) est configuré pour mesurer la densité (p) de l'échantillon de gaz (Gp), et
dans lequel l'agencement de capteurs (50) est configuré pour
déterminer les concentrations des trois composants gazeux en utilisant

- les densités prédéfinies des composants gazeux,
- les conductivités thermiques prédéfinies des composants gazeux,
- la conductivité thermique ($\lambda$) mesurée de l'échantillon de gaz (Gp),
- la conductivité thermique à modulation magnétique ($\lambda_{2f}$) mesurée de l'échantillon de gaz (Gp) et
- la densité (p) mesurée de l'échantillon de gaz (Gp).

**2.** Agencement de capteurs (50) selon la revendication 1,
**caractérisé en ce que**

l'agencement de capteurs (50) comprend une unité chauffante (8, 66),
dans lequel l'unité chauffante (8, 66) est configurée pour amener de l'énergie thermique à un échantillon de gaz (Gp) dans la chambre de mesure (2),
dans lequel l'agencement de capteurs (50) est configuré pour

- mesurer une grandeur de détection électrique (U) qui est en corrélation avec la conductivité thermique ($\lambda$)

de l'échantillon de gaz (Gp), et

- à l'aide d'un filtrage de la grandeur de détection électrique (U), déduire un signal oscillant (75) qui oscille en fonction de l'intensité de champ magnétique, et un autre signal (76) dont l'évolution temporelle ne dépend pas de l'intensité de champ magnétique oscillante, et

dans lequel l'agencement de capteurs (50) est en outre configuré pour

- mesurer la conductivité thermique à modulation magnétique ($\lambda_{2f}$) de l'échantillon de gaz (Gp) en fonction du signal oscillant (75), en particulier utiliser le signal oscillant (75) comme conductivité thermique à modulation magnétique ($\lambda_{2f}$) de l'échantillon de gaz (Gp), et
- mesurer la conductivité thermique ($\lambda$) de l'échantillon de gaz (Gp) en fonction de l'autre signal (76), en particulier utiliser l'autre signal (76) comme conductivité thermique ($\lambda$).

3. Agencement de capteurs (50) selon l'une des revendications précédentes,
**caractérisé en ce que**

le capteur de densité comprend un résonateur de flexion (59, 60) comportant un corps vibrant (80, 61.1, 61.2), dans lequel l'agencement de capteurs (50) est configuré pour guider l'échantillon de gaz (Gp) à travers le corps vibrant (80, 61.1, 61.2) ou le long de celui-ci, dans lequel le résonateur de flexion (59, 60) est configuré pour

- faire vibrer le corps vibrant (80, 61.1, 61.2) et
- mesurer la fréquence propre du corps vibrant (80, 61.1, 61.2) en train de vibrer, et

dans lequel l'agencement de capteurs (50) est configuré pour déterminer la densité (p) de l'échantillon de gaz (Gp) en fonction de la fréquence propre mesurée du corps vibrant (80, 61.1, 61.2).

4. Agencement de ventilation (200) pour la respiration artificielle d'un patient (P),

dans lequel l'agencement de ventilation (200) comprend

- un appareil médical (1), en particulier un appareil de ventilation,
- une unité de couplage côté patient (21),
- un appareil de commande de ventilation (35) et
- un agencement de capteurs (50) selon l'une des revendications précédentes,

dans lequel l'unité de couplage côté patient (21) peut être reliée ou est reliée au moins temporairement au patient (Pt), dans lequel l'agencement de ventilation (200) est conçu pour transporter un mélange gazeux (Gg) respirable depuis l'appareil médical (1) jusqu'à l'unité de couplage côté patient (21), dans lequel une plage de consigne pour la concentration [$\xi(O_2)$] d'un composant gazeux ($O_2$) dans le mélange gazeux (Gg) est prédéfinie, dans lequel, de préférence, ledit composant gazeux est le gaz paramagnétique, dans lequel l'agencement de ventilation (200) est configuré pour

- dériver un échantillon de gaz (Gp) du mélange gazeux (Gg) transporté jusqu'à l'unité de couplage côté patient (21) et
- conduire l'échantillon de gaz (Gp) dérivé vers l'agencement de capteurs (50), et

dans lequel l'agencement de capteurs (50) est configuré pour mesurer la concentration [$\xi(O_2)$] réelle du composant gazeux ($O_2$), pour lequel la plage de consigne est prédéfinie, dans l'échantillon de gaz (Gp) dérivé.

5. Agencement de ventilation (200) selon la revendication 4,
**caractérisé en ce que**

l'appareil de commande de ventilation (35) est configuré pour

- réguler la concentration [$\xi(O_2)$] du composant gazeux ($O_2$), pour lequel la plage de consigne est prédéfinie, dans le mélange gazeux (Gg) qui est transporté jusqu'à l'unité de couplage côté patient (21) et
- utiliser la concentration du composant gazeux [ξ(O$_2$)] mesurée pour la régulation, et

dans lequel un objectif de régulation est, lors de la régulation, que la concentration de composant gazeux [ξ(O$_2$)] réelle dans le mélange gazeux (Gg) reste dans la plage de consigne prédéfinie.

6. Procédé de mesure permettant de mesurer la concentration respective de trois composants gazeux d'un échantillon de gaz (Gp),

dans lequel la densité respective et la conductivité thermique respective de chacun des trois composants gazeux sont prédéfinies,
dans lequel un composant gazeux (O$_2$) des trois composants gazeux est un gaz paramagnétique et
dans lequel le procédé de mesure comprend les étapes selon lesquelles

- la conductivité thermique de l'échantillon de gaz (Gp) est mesurée et
- la densité de l'échantillon de gaz (Gp) est mesurée,

dans lequel le procédé de mesure comprend les étapes supplémentaires selon lesquelles

- l'échantillon de gaz (Gp) est conduit vers une chambre de mesure (2),
- un champ magnétique comportant une intensité de champ magnétique oscillante est appliqué à la chambre de mesure (2) et
- une conductivité thermique à modulation magnétique ($\lambda_{2f}$) de l'échantillon de gaz (Gp) est mesurée dans la chambre de mesure (2),

dans lequel la conductivité thermique à modulation magnétique ($\lambda_{2f}$) est la fraction de la conductivité thermique de l'échantillon de gaz (Gp) qui oscille en fonction de l'intensité du champ magnétique, et
dans lequel l'étape supplémentaire est réalisée, selon laquelle
les concentrations des trois composants gazeux sont déterminées en utilisant

- les densités prédéfinies des composants gazeux,
- les conductivités thermiques prédéfinies des composants gazeux,
- la conductivité thermique ($\lambda$) mesurée de l'échantillon de gaz (Gp),
- la conductivité thermique à modulation magnétique ($\lambda_{2f}$) mesurée de l'échantillon de gaz (Gp) et
- la densité (p) mesurée de l'échantillon de gaz (Gp).

7. Procédé de mesure selon la revendication 6,
**caractérisé en ce que**

la conductivité thermique de l'échantillon de gaz (Gp) dans la chambre de mesure (2) est mesurée en tant que conductivité thermique ($\lambda$) et
le procédé de mesure comprend les étapes supplémentaires selon lesquelles

- une unité chauffante (8, 66) est chauffée,
- l'unité chauffante (8, 66) chauffée amène de l'énergie thermique à l'échantillon de gaz (Gp) dans la chambre de mesure (2),
- une grandeur de détection électrique (U) de l'unité chauffante (8, 66) chauffée est mesurée,

dans lequel la grandeur de détection (U) est en corrélation avec la conductivité thermique ($\lambda$) de l'échantillon de gaz (Gp), et

- au moyen d'un filtrage de la grandeur de détection électrique (U), un signal oscillant (75) est dérivé, lequel oscille en fonction de l'intensité de champ magnétique, et un autre signal (76) est dérivé, dont l'évolution temporelle ne dépend pas de l'intensité de champ magnétique oscillante,

dans lequel

- la conductivité thermique à modulation magnétique ($\lambda_{2f}$) de l'échantillon de gaz (Gp) est mesurée en fonction du signal oscillant (75),

en particulier, le signal oscillant (75) est utilisé comme conductivité thermique à modulation magnétique ($\lambda_{2f}$) de l'échantillon de gaz (Gp), et

- la conductivité thermique ($\lambda$) de l'échantillon de gaz (Gp) est mesurée en fonction de l'autre signal (76),

en particulier, l'autre signal (76) est utilisé comme conductivité thermique ($\lambda$).

**8.** Procédé de mesure selon la revendication 6 ou la revendication 7,
**caractérisé en ce que**

le procédé de mesure comprend les étapes supplémentaires selon lesquelles

- l'échantillon de gaz (Gp) est conduit à travers un corps vibrant (80, 61.1, 61.2) d'un résonateur de flexion (59, 60) ou le long de celui-ci,
- le corps vibrant (80, 61.1, 61.2) est mis en vibration et
- la fréquence propre du corps vibrant (80, 61.1, 61.2) en train de vibrer est mesurée,

dans lequel la densité de l'échantillon de gaz (Gp) est déterminée en fonction de la fréquence propre mesurée du corps vibrant (80, 61.1, 61.2).

**9.** Procédé de mesure selon l'une des revendications 6 à 8,
**caractérisé en ce que**

la conductivité thermique, la conductivité thermique à modulation magnétique et/ou la densité de l'échantillon de gaz (Gp) dépendent en outre de la concentration d'un quatrième composant gazeux,
dans lequel la densité du quatrième composant gazeux est prédéfinie et
dans lequel le procédé de mesure comprend les étapes supplémentaires selon lesquelles

- la concentration du quatrième composant gazeux est mesurée ou déterminée d'une autre manière et
- lors de l'étape consistant à déterminer la concentration respective des trois composants gazeux, la concentration mesurée du quatrième composant gazeux est en outre utilisée.

**10.** Procédé de mesure selon la revendication 9,
**caractérisé en ce que**

l'étape consistant à mesurer la concentration du quatrième composant gazeux
comprend les étapes selon lesquelles

- une source de rayonnement émet un rayonnement électromagnétique ou un son,
- le rayonnement électromagnétique émis ou le son émis traverse l'échantillon de gaz (Gp) et frappe un détecteur,
- le détecteur mesure l'intensité du rayonnement électromagnétique incident ou du son incident et
- le détecteur génère un signal en fonction de l'intensité mesurée,

dans lequel la concentration du quatrième composant gazeux est mesurée en fonction du signal généré par le détecteur,
en particulier, le signal généré par le détecteur est utilisé comme concentration du quatrième composant gazeux.

**11.** Procédé de mesure selon l'une des revendications 6 à 10,
**caractérisé en ce que**

trois relations fonctionnelles, trois mesures de distance et une fonction cible sont prédéfinies,
dans lequel la première relation fonctionnelle décrit la conductivité thermique ($\lambda$) de l'échantillon de gaz (Gp) en fonction des concentrations des trois composants gazeux,
dans lequel la deuxième relation fonctionnelle décrit la conductivité thermique à modulation magnétique ($\lambda_{2f}$) de l'échantillon de gaz (Gp) en fonction des concentrations des trois composants gazeux,

dans lequel la troisième relation fonctionnelle décrit la densité (p) de l'échantillon de gaz (Gp) en fonction des concentrations des trois composants gazeux,

dans lequel la première mesure de distance décrit une mesure d'une distance entre la conductivité thermique ($\lambda$) mesurée de l'échantillon de gaz (Gp) et la conductivité thermique déduite à l'aide de la première relation fonctionnelle,

dans lequel la deuxième mesure de distance décrit une mesure d'une distance entre la conductivité thermique à modulation magnétique ($\lambda_{2f}$) mesurée de l'échantillon de gaz (Gp) et la conductivité thermique à modulation magnétique déduite à l'aide de la deuxième relation fonctionnelle,

dans lequel la troisième mesure de distance décrit une mesure d'une distance entre la densité (p) mesurée de l'échantillon de gaz (Gp) et la densité déduite à l'aide de la troisième relation fonctionnelle,

dans lequel la fonction cible est une agrégation, en particulier une moyenne pondérée, des trois mesures de distance et

dans lequel le procédé de mesure comprend les étapes supplémentaires selon lesquelles

- les trois valeurs mesurées pour la conductivité thermique ($\lambda$), la conductivité thermique à modulation magnétique ($\lambda_{2f}$) et la densité de l'échantillon de gaz (p) sont utilisées dans la fonction cible et
- un triplet de valeurs est calculé pour les concentrations des trois composants gazeux de telle sorte que la fonction cible est minimisée.

**12.** Procédé de mesure selon l'une des revendications 6 à 11,
**caractérisé en ce que**
les trois composants gazeux, dont les concentrations sont mesurées dans l'échantillon de gaz (Gp) dérivé, sont l'oxygène ($O_2$), un agent anesthésique et l'argon.

**13.** Procédé de ventilation pour la respiration artificielle d'un patient (P),

dans lequel le patient (P) est relié à une unité de couplage côté patient (21) et
dans lequel le procédé de ventilation comprend les étapes réalisées automatiquement selon lesquelles

- un mélange gazeux (Gg) respirable est transporté depuis un appareil médical (1), en particulier depuis un appareil de ventilation, jusqu'à l'unité de couplage côté patient (21),
- une plage de consigne pour la concentration [$\xi(O_2)$] d'un composant gazeux ($O_2$) dans le mélange gazeux (Gg) est prédéfinie,
- un échantillon de gaz (Gp) est dérivé du mélange gazeux (Gg) transporté jusqu'à l'unité de couplage côté patient (21),
- la concentration [$\xi(O_2)$] réelle du composant gazeux ($O_2$) pour lequel la plage de consigne est prédéfinie est mesurée,

dans lequel, pour la mesure de la concentration de composant gazeux [$\xi(O_2)$] réelle, un procédé de mesure selon l'une des revendications 6 à 12 est utilisé et

dans lequel le composant gazeux pour lequel la plage de consigne est prédéfinie est l'un des trois composants gazeux dont la concentration est mesurée, de préférence le gaz paramagnétique,

- dans lequel, de préférence, la concentration [$\xi(O_2)$] réelle du composant gazeux ($O_2$) dans le mélange gazeux (Gg) est régulée avec l'objectif de régulation selon lequel la concentration réelle d'oxygène se situe dans la plage de consigne prédéfinie,

dans lequel la concentration de composant gazeux [$\xi(O_2)$] réelle mesurée dans l'échantillon de gaz (Gp) dérivé est utilisée pour la régulation.

**14.** Procédé de ventilation selon la revendication 13,
**caractérisé en ce que**

le mélange gazeux (Gg) est produit en utilisant l'air ambiant,
dans lequel, lors de la production du mélange gazeux (Gg), la concentration d'oxygène est augmentée et
dans lequel les trois composants gazeux dont les concentrations sont mesurées dans l'échantillon de gaz (Gp) dérivé
sont l'oxygène ($O_2$), un agent anesthésique et l'argon.

**15.** Procédé de ventilation selon la revendication 13 ou la revendication 14,
**caractérisé en ce que**

le mélange gazeux (Gg) est transporté à travers une unité de guidage de fluide (32) depuis l'appareil médical (1) jusqu'à l'unité de couplage côté patient (21) et
la détection de l'événement selon lequel au moins un composant gazeux dans le mélange gazeux (Gg) transporté se trouve en dehors de la plage de consigne prédéfinie pour ce composant gazeux
déclenche l'étape consistant à rincer l'unité de guidage de fluide (32).

FIG. 1

FIG. 2

EP 4 538 701 B1

**FIG. 3**

FIG. 4

FIG. 5

FIG. 6

EP 4 538 701 B1

**FIG. 7A**

**FIG. 7B**

EP 4 538 701 B1

FIG. 8

EP 4 538 701 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102021126106 A1

- GB 2355806 A

**In der Beschreibung aufgeführte Nicht-Patentliteratur**

- **TH. VOGLHUBER-BRUNNMEIER et al.** Fluid Sensing Using Quartz Tuning Forks - Measurement Technology and Applications. *Sensors*, 2019, vol. 19 (10)